# EUROPEAN PATENT APPLICATION

(11) **EP 2 835 374 A1**
(43) Date of publication of application: **11.02.2015**
(21) Application number: 13382320.3
(22) Date of filing: 06.08.2013
(51) Int. Cl.: C07F 15/00, C07B 37/00, C07F 1/08, C07F 9/50, C07C 17/263, C07C 17/32, C07C 21/18, C07C 22/08, C07C 25/13, C07C 25/22

(54) **Pentafluoroethylating compositions**

(71) Applicant: Fundació Institut Català d'Investigació Química, 43007 Tarragona (ES)
(72) Inventor: Grushin, Vladimir, 43007 Tarragona (ES); Lishchynskyi, Anton, 43001 Tarragona (ES)
(74) Representative: Watson, Robert James

(57) **Abstract**

The present invention relates to pentafluoroethylating compositions, processes for obtaining them, and their use in pentafluoroethylation reactions.

## Description

### Field of the invention

The present invention relates to pentafluoroethylating compositions, processes for obtaining them, and their use in pentafluoroethylation reactions.

### Background of the invention

Numerous organic compounds bearing a trifluoromethyl group are valuable as pharmaceuticals and agrochemicals. Organic compounds bearing a pentafluoroethyl group are much less common, but are in demand for the development of biologically active compounds and materials [US4837364, US20120190660A1] and have been reported to exhibit properties that are superior to those of their trifluoromethyl analogues [Andrzejewska 2002, Johansson 2003].

Unlike trifluoromethyl aromatic derivatives, pentafluoroethylated aromatic compounds cannot be made by the Swarts reaction, and, as a result, are much less accessible.

Some synthetic methods to produce such compounds have been developed, which use copper reagents bearing the pentafluoroethyl group. Such copper reagents can be generated in situ from pentafluoroethyl iodide and copper, by decarboxylation of sodium, potassium, or methyl perfluoropropionate in the presence of Cu(I), or via C₂F₅ transfer from C₂F₅SiMe₃ to copper.

Trifluoromethylation with CuCF₃ at elevated temperatures is often accompanied by side pentafluoroethylation reactions due to the in situ formation of CuC₂F₅ via α-fluorine-elimination and CF₂ insertion into the Cu-CF₃ bond. This can often lead to a mixture of products bearing C₂F₅ and other perfluoroalkyl substituents.

Sodium and potassium pentafluoropropionates in the presence of Cul have been used for the pentafluoroethylation of aryl halides, requiring temperatures up to 170°C for the decarboxylation to proceed [Carr 1988, Freskos 1988]. Such temperatures limit the scope of substrates to which it can be applied.

Pentafluoroethylation of iodoarenes has been performed with C₂F₅SiMe₃ and CuI, in the presence of KF at 60°C [Urata 1991, JPH03218325]. The source of the perfluoroalkyl group in this method is expensive. Furthermore, this reaction is not "atom-economical. Heating CF₂Br₂ or C₂F₂BrCl with Cu powder in DMF leads to a mixture of perfluoroalkyl copper reagents CF₃(CF₂)ₙCu with n being up to 16. The distribution can be controlled by temperature and other factors. For example, CF₂BrCl at 70°C favours the generation of C₂F₅Cu with 80% selectivity [US4582921]. However, the obtained compound may suffer from low stability and the use of the perfluoroethylcopper thus obtained in perfluoroethylation reactions may not be selective.

A similar approach to the formation of C₂F₅Cu can be carried out starting from Zn(CF₃)Br•2DMF, mixing with CuBr in DMF, followed by heating to generate the pentafluoroethyl copper species which can be used in situ [Kremlev 2010].

Another approach to the formation of C₂F₅Cu species involves the initial formation of a pentafluoroethyl zinc reagent from pentafluoroethane by using a zinc base, followed by transmetallation with a copper halide [Popov 2011]. This approach was developed as the authors considered most perfluoroalkyl metals to be unstable, and only mercury, cadmium, bismuth, thallium and zinc perfluoroalkyls to be stable. The use of mercury, cadmium and thallium was dismissed due to toxicity issues, and bismuth was not used as bismuth halides are photolytically and thermally unstable.

On the other hand, Zanardi et al. reported a method for the direct cupration of fluoroform (HCF₃) using a cuprating agent formed from a copper (I) source and a base, said cuprating agent being preferably a dialkoxycuprate. However, the authors are silent about the cupration reaction of other mono- or polyhydrogenated perfluoroalkanes [Zanardi 2011].

No one has ever disclosed preparation of a pentafluoroethyl copper compound directly from a copper reagent and pentafluoroethane.

It is desirable to develop a method for obtaining a pentafluoroethyl copper composition directly from a copper reagent and pentafluoroethane, which is useful for synthetic transformations leading to the introduction of the pentafluoroethyl group into other molecules in order to make useful materials.

### Description of the invention

The inventors carried out a series of experiments in which the cuprating reagent reported by Zanardi was contacted with one of the following compounds: CF₃(CF₂)₃H, CF₃(CF₂)₅H, CF₃(CF₂)₇H, HCF₂(CF₂)₄CF₂H, and (CF₃)₂CFH. In all the cases, the inventors found that complex mixtures of fluorinated products and KF and/or KHF₂ were obtained. It was totally unexpected to the inventors when, after the invariably negative results observed with all of these polyfluoroalkanes, pentafluoroethane, C₂F₅H, underwent clean conversion to a C₂F₅Cu derivative under the same conditions.

Thus, the first aspect of the invention provides a method of obtaining a pentafluoroethylating composition which comprises reacting a cuprating agent comprising the [Cu(OR¹)(OR²)] moiety with pentafluoroethane, wherein:
Cu is in the oxidation state of +1; and
R¹ is C₁₋₂₀ alkyl or C₅₋₂₀ aryl, wherein C₁₋₂₀ alkyl is optionally substituted with one or more groups selected from: C₅₋₂₀ aryl, C₁₋₂₀ alkoxy and C₅₋₂₀ aryloxy, and wherein C₅₋₂₀ aryl is optionally substituted with one or more groups selected from: C₁₋₂₀ alkyl, C₁₋₂₀ alkoxy, C₅₋₂₀ aryl, C₅₋₂₀ aryloxy, halo, nitro and cyano; and
R² is C₁₋₂₀ alkyl or C₅₋₂₀ aryl, wherein C₁₋₂₀ alkyl is optionally substituted with one or more groups selected from: C₅₋₂₀ aryl, C₁₋₂₀ alkoxy and C₅₋₂₀ aryloxy, and wherein C₅₋₂₀ aryl is optionally substituted with one or more groups selected from: C₁₋₂₀ alkyl, C₁₋₂₀ alkoxy, C₅₋₂₀ aryl, C₅₋₂₀ aryloxy, halo, nitro and cyano.

The cuprating agent used in the first aspect of the invention is described in WO2012/113726, which is herein incorporated by reference.

Thus, the first aspect also comprises the initial step of contacting a copper (I) source and a base in the presence of a solvent to form a cuprating reagent, where the base is a compound of formula **I** or a mixture of compounds of formula **I**:

M-OR¹ (**I**)

wherein M is selected from the group consisting of lithium, sodium, potassium, rubidium, cesium, calcium, barium and magnesium; and
R¹ is as defined above.

The second aspect of the present invention relates to a pentafluoroethylating composition obtainable by the process defined above.

The second aspect of the present invention also provides a pentafluroethylating composition which comprises the [(C₂F₅)Cu(OR¹)] moiety, wherein R¹ is as defined in the first aspect.

The third aspect of the present invention relates to the use of the pentafluoroethylating composition of the second aspect of the invention in the transfer of a pentafluoroethyl group to an electrophile, a terminal alkyne, or to the carbon atom of an organoboron compound.

The fourth aspect of the present invention relates to the use of the pentafluoroethylating composition of the second aspect of the invention in the preparation of further pentafluoroethyl copper complexes.

The fifth aspect of the invention relates to pentafluoroethyl copper complexes obtained according to the fourth aspect.

### Definitions

The term "C₅₋₂₀ aryl" in the present application means a monovalent moiety obtained by removing a hydrogen atom from an aromatic ring of an aromatic compound, which moiety has 5-20 ring atoms. Each ring has 5 to 7 ring atoms.

In this context, the prefixes (e.g. C₅₋₂₀, C₅₋₇, C₅₋₆, etc.) denote the number of ring atoms, or range of number of ring atoms, whether carbon atoms or heteroatoms. For example, the term "C₅₋₆ aryl" as used herein, pertains to an aryl group having 5 or 6 ring atoms.

The ring atoms may be all carbon atoms, as in "carboaryl groups". Examples of carboaryl groups include, but are not limited to, those derived from benzene (i.e. phenyl) (C₆), naphthalene (C₁₀), azulene (C₁₀), anthracene (C₁₄), phenanthrene (C₁₄), naphthacene (C₁₈), and pyrene (C₁₆).

Examples of aryl groups which comprise fused rings, at least one of which is an aromatic ring, include, but are not limited to, groups derived from indane (e.g. 2,3-dihydro-1H-indene) (C₉), indene (Cg), isoindene (C₉), tetraline (1,2,3,4-tetrahydronaphthalene (C₁₀), acenaphthene (C₁₂), fluorene (C₁₃), phenalene (C₁₃), acephenanthrene (C₁₅), and aceanthrene (C₁₆).

Alternatively, the ring atoms may include one or more heteroatoms, as in "heteroaryl groups". Examples of monocyclic heteroaryl groups include, but are not limited to, those derived from:
N₁: pyrrole (azole) (C₅), pyridine (azine) (C₆);
O₁: furan (oxole) (C₅);
S₁: thiophene (thiole) (C₅);
N₁O₁: oxazole (C₅), isoxazole (C₅), isoxazine (C₆);
N₂O₁: oxadiazole (furazan) (C₅);
N₃O₁: oxatriazole (C₅);
N₁S₁: thiazole (C₅), isothiazole (C₅);
N₂: imidazole (1,3-diazole) (C₅), pyrazole (1,2-diazole) (C₅), pyridazine (1,2-diazine) (C₆), pyrimidine (1,3-diazine) (C₆) (e.g., cytosine, thymine, uracil), pyrazine (1,4-diazine) (C₆);
N₃: triazole (C₅), triazine (C₆); and,
N₄: tetrazole (C₅).

Examples of heteroaryls which comprise fused rings, include, but are not limited to:
C₉(with 2 fused rings) derived from benzofuran (O₁), isobenzofuran (O₁), indole (N₁), isoindole (N₁), indolizine (N₁), indoline (N₁), isoindoline (N₁), purine (N₄) (e.g., adenine, guanine), benzimidazole (N₂), indazole (N₂), benzoxazole (N₁O₁), benzisoxazole (N₁O₁), benzodioxole (O₂), benzofurazan (N₂O₁), benzotriazole (N₃), benzothiofuran (S₁), benzothiazole (N₁S₁), benzothiadiazole (N₂S);
C₁₀ (with 2 fused rings) derived from chromene (O₁), isochromene (O₁), chroman (O₁), isochroman (O₁), benzodioxan (O₂), quinoline (N₁), isoquinoline (N₁), quinolizine (N₁), benzoxazine (N₁O₁), benzodiazine (N₂), pyridopyridine (N₂), quinoxaline (N₂), quinazoline (N₂), cinnoline (N₂), phthalazine (N₂), naphthyridine (N₂), pteridine (N₄);
C₁₁ (with 2 fused rings) derived from benzodiazepine (N₂);
C₁₃ (with 3 fused rings) derived from carbazole (N₁), dibenzofuran (O₁), dibenzothiophene (S₁), carboline (N₂), perimidine (N₂), pyridoindole (N₂); and,
C₁₄ (with 3 fused rings) derived from acridine (N₁), xanthene (O₁), thioxanthene (S₁), oxanthrene (O₂), phenoxathiin (O₁S₁), phenazine (N₂), phenoxazine (N₁O₁), phenothiazine (N₁S₁), thianthrene (S₂), phenanthridine (N₁), phenanthroline (N₂), phenazine (N₂).

Thus, the term "C₅₋₇ aryl" means a 5-, 6-, and 7-membered aryl group as defined above, for example, phenyl, pyrollyl, pyridinyl, furanyl, thiophenyl, oxazolyl, isoxazolyl, isoxazinyl, and furazanyl,

The term "alkyl" in the present application means aliphatic saturated or unsaturated (e.g. partially unsaturated, fully unsaturated) groups including linear, cyclic or branched alkyl groups. Thus, the term "alkyl" includes the sub-classes alkenyl, alkynyl, cycloalkyl, etc., discussed below.

C₁₋₂₀ alkyl: The term "C₁₋₂₀ alkyl" as used herein, pertains to a monovalent moiety obtained by removing a hydrogen atom from a carbon atom of a hydrocarbon compound having from 1 to 20 carbon atoms, which may be aliphatic or alicyclic, and which may be saturated or unsaturated (e.g. partially unsaturated, fully unsaturated).

Examples of saturated alkyl groups include, but are not limited to, methyl (C₁), ethyl (C₂), propyl (C₃), butyl (C₄), pentyl (C₅), hexyl (C₆) and heptyl (C₇).

Examples of saturated linear alkyl groups include, but are not limited to, methyl (C₁), ethyl (C₂), n-propyl (C₃), n-butyl (C₄), n-pentyl (amyl) (C₅), n-hexyl (C₆) and n-heptyl (C₇).

Examples of saturated branched alkyl groups include iso-propyl (C₃), iso-butyl (C₄), sec-butyl (C₄), tert-butyl (C₄), iso-pentyl (C₅), and neo-pentyl (C₅).

C₂₋₂₀ Alkenyl: The term "C₂₋₂₀ alkenyl" as used herein, pertains to an alkyl group having one or more carbon-carbon double bonds.

Examples of unsaturated alkenyl groups include, but are not limited to, ethenyl (vinyl, - CH=CH₂), 1-propenyl (-CH=CH-CH₃), 2-propenyl (allyl, -CH-CH=CH₂), isopropenyl (1-methylvinyl, -C(CH₃)=CH₂), butenyl (C₄), pentenyl (C₅), and hexenyl (C₆).

C₂₋₂₀ alkynyl: The term "C₂₋₂₀ alkynyl" as used herein, pertains to an alkyl group having one or more carbon-carbon triple bonds.

Examples of unsaturated alkynyl groups include, but are not limited to, ethynyl (-C≡CH) and 2-propynyl (propargyl, -CH₂-C≡CH).

C₃₋₂₀ cycloalkyl: The term "C₃₋₂₀ cycloalkyl" as used herein, pertains to an alkyl group which is also a cyclyl group; that is, a monovalent moiety obtained by removing a hydrogen atom from an alicyclic ring atom of a cyclic hydrocarbon (carbocyclic) compound, which moiety has from 3 to 7 carbon atoms, including from 3 to 7 ring atoms.

Examples of cycloalkyl groups include, but are not limited to, those derived from:
saturated monocyclic hydrocarbon compounds:
   cyclopropane (C₃), cyclobutane (C₄), cyclopentane (C₅), cyclohexane (C₆), cycloheptane (C₇), methylcyclopropane (C₄), dimethylcyclopropane (C₅), methylcyclobutane (C₅),
   dimethylcyclobutane (C₆), methylcyclopentane (C₆), dimethylcyclopentane (C₇) and
   methylcyclohexane (C₇);
unsaturated monocyclic hydrocarbon compounds:
   cyclopropene (C₃), cyclobutene (C₄), cyclopentene (C₅), cyclohexene (C₆),
   methylcyclopropene (C₄), dimethylcyclopropene (C₅), methylcyclobutene (C₅),
   dimethylcyclobutene (C₆), methylcyclopentene (C₆), dimethylcyclopentene (C₇) and methylcyclohexene (C₇); and
saturated polycyclic hydrocarbon compounds:
   norcarane (C₇), norpinane (C₇), norbornane (C₇).

The terms "*tert*-Bu", "*t*-Bu", "Bu-*t*" and "*tert*-butyl" are interchangeable and are used to refer to the *tert*-butyl group of formula -C(CH₃)₃.

C₃₋₂₀ heterocyclyl: The term "C₃₋₂₀ heterocyclyl" as used herein, pertains to a monovalent moiety obtained by removing a hydrogen atom from a ring atom of a heterocyclic compound, which moiety has from 3 to 20 ring atoms, of which from 1 to 10 are ring heteroatoms. Preferably, each ring has from 3 to 7 ring atoms, of which from 1 to 4 are ring heteroatoms.

In this context, the prefixes (e.g. C₃₋₂₀, C₃₋₇, C₅₋₆, etc.) denote the number of ring atoms, or range of number of ring atoms, whether carbon atoms or heteroatoms. For example, the term "C₅₋₆heterocyclyl", as used herein, pertains to a heterocyclyl group having 5 or 6 ring atoms.

Examples of monocyclic heterocyclyl groups include, but are not limited to, those derived from:
N₁: aziridine (C₃), azetidine (C₄), pyrrolidine (tetrahydropyrrole) (C₅), pyrroline (e.g., 3-pyrroline, 2,5-dihydropyrrole) (C₅), 2H-pyrrole or 3H-pyrrole (isopyrrole, isoazole) (C₅),
piperidine (C₆), dihydropyridine (C₆), tetrahydropyridine (C₆), azepine (C₇);
O₁: oxirane (C₃), oxetane (C₄), oxolane (tetrahydrofuran) (C₅), oxole (dihydrofuran) (C₅), oxane (tetrahydropyran) (C₆), dihydropyran (C₆), pyran (C₆), oxepin (C₇);
S₁: thiirane (C₃), thietane (C₄), thiolane (tetrahydrothiophene) (C₅), thiane (tetrahydrothiopyran) (C₆), thiepane (C₇);
O₂: dioxolane (C₅), dioxane (C₆), and dioxepane (C₇);
O₃: trioxane (C₆);
N₂: imidazolidine (C₅), pyrazolidine (diazolidine) (C₅), imidazoline (C₅), pyrazoline (dihydropyrazole) (C₅), piperazine (C₆);
N₁O₁: tetrahydrooxazole (C₅), dihydrooxazole (C₅), tetrahydroisoxazole (C₅), dihydroisoxazole (C₅), morpholine (C₆), tetrahydrooxazine (C₆), dihydrooxazine (C₆), oxazine (C₆);
N₁S₁: thiazoline (C₅), thiazolidine (C₅), thiomorpholine (C₆);
N₂O₁: oxadiazine (C₆);
O₁S₁: oxathiole (C₅) and oxathiane (thioxane) (C₆); and,
N₁O₁S₁: oxathiazine (C₆).

Examples of substituted monocyclic heterocyclyl groups include those derived from saccharides, in cyclic form, for example, furanoses (C₅), such as arabinofuranose, lyxofuranose, ribofuranose, and xylofuranose, and pyranoses (C₆), such as allopyranose, altropyranose, glucopyranose, mannopyranose, gulopyranose, idopyranose, galactopyranose, and talopyranose.

The above groups, whether alone or part of another substituent, may themselves optionally be substituted with one or more groups selected from themselves and the additional substituents listed below, unless specified otherwise.
Halo: -F, -Cl, -Br, and -I
Hydroxy: -OH
Oxo (keto, -one): =O
Thione (thioketone): =S
Formyl (carbaldehyde, carboxaldehyde): -C(=O)H
Carboxy (carboxylic acid): -C(=O)OH
Thiocarboxy (thiocarboxylic acid): -C(=S)SH
Thiolocarboxy (thiolocarboxylic acid): -C(=O)SH
Thionocarboxy (thionocarboxylic acid): -C(=S)OH
Imidic acid: -C(=NH)OH
Hydroxamic acid: -C(=NOH)OH
Guanidino: -NH-C(=NH)NH₂
Nitro: -NO₂
Nitroso: -NO
Azido: -N₃
Cyano (nitrile, carbonitrile): -CN
Isocyano: -NC
Cyanato: -OCN
Isocyanato: -NCO
Thiocyano (thiocyanato): -SCN
Isothiocyano (isothiocyanato): -NCS
Sulfhydryl (thiol, mercapto): -SH
Sulfinic acid (sulfino): -S(=O)OH, -SO₂H
Sulfonic acid (sulfo): -S(=O)₂OH, -SO₃H
Phosphonic acid (phosphono): -P(=O)(OH)₂
Phosphoric acid (phosphonooxy): -OP(=O)(OH)₂.
Phosphorous acid: -OP(OH)₂
Tetrazolyl: a five membered aromatic ring having four nitrogen atoms and one carbon atom,

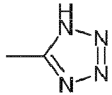

In the following substituent groups, R is selected from, for example, a C₁₋₇ alkyl group, a C₃₋₂₀ heterocyclyl group, and a C₅₋₂₀ aryl group, preferably a C₁₋₇ alkyl group:
Ether: -OR
Acyl (keto): -C(=O)R
Hemiacetal: -CH(OH)(OR)
Hemiketal: -CR(OH)(OR)
Ester (carboxylate, carboxylic acid ester, oxycarbonyl): -C(=O)OR
Acyloxy (reverse ester): -OC(=O)R
Oxycarboyloxy: -OC(=O)OR
Thioether (sulfide): -SR
Disulfide: -SS-R
Sulfine (sulfinyl, sulfoxide): -S(=O)R
Sulfone (sulfonyl): -S(=O)₂R
Amidine (amidino): -C(=NR)NR₂
Sulfinate (sulfinic acid ester): -S(=O)OR
Sulfonate (sulfonic acid ester): -S(=O)₂OR
Sulfinyloxy: -OS(=O)R
Sulfonyloxy: -OS(=O)₂R
Sulfate: -OS(=O)₂OR
Phosphinyl (phosphine oxide): -P(=O)R₂
Alkoxy: -OR, wherein R is an alkyl group, for example, a C₁₋₇alkyl group. Examples of C₁₋₇ alkoxy groups include, but are not limited to, -OMe (methoxy), -OEt (ethoxy), -O(nPr) (n-propoxy), -O(iPr) (isopropoxy), -O(nBu) (n-butoxy), -O(sBu) (sec-butoxy), -O(iBu) (isobutoxy), and -O(tBu) (tert-butoxy).

In the following substituent groups, R^{A} and R^{B} are independently, for example, hydrogen, a C₁₋₇ alkyl group, a C₃₋₂₀ heterocyclyl group, or a C₅₋₂₀ aryl group, preferably H or a C₁₋₇ alkyl group, or, in the case of a "cyclic" group, R^{A} and R^{B}, taken together with the nitrogen atom to which they are attached, form a heterocyclic ring having from 4 to 8 ring atoms:
Amino: -NR^{A}R^{B}
Amido (carbamoyl, carbamyl, aminocarbonyl, carboxamide): -C(=O)NR^{A}R^{B} Thioamido (thiocarbamyl): -C(=S)NR^{A}R^{B}
Aminocarbonyloxy: -OC(=O)NR^{A}R^{B}
Sulfamyl (sulfamoyl; sulfinic acid amide; sulfinamide): -S(=O)NR^{A}R^{B} Sulfonamido (sulfinamoyl; sulfonic acid amide; sulfonamide): -S(=O)₂NR^{A}R^{B}

In the following substituent groups, R^{A} and R^{B} are independently, for example, a C₁₋₇alkyl group, a C₃₋₂₀ heterocyclyl group, or a C₅₋₂₀ aryl group, preferably a C₁₋₇ alkyl group, or, in the case of a "cyclic" group, R^{A} and R^{B}, taken together with the two oxygen atoms to which they are attached, and the carbon atoms to which they are attached, form a heterocyclic ring having from 4 to 8 ring atoms:
Acetal: -CH(OR^{A})(OR^{B})
Ketal: -CR(OR^{A})(OR^{B})

In the following substituent groups, R is selected from, for example, H, a C₁₋₇ alkyl group, a C₃₋₂₀heterocyclyl group, and a C₅₋₂₀ aryl group, preferably a C₁₋₇alkyl group:
Sulfamino: -NRS(=O)₂OH
Imino (imine): =NR
Phosphino (phosphine): -PR₂
Phosphonate (phosphono ester): -P(=O)(OR)₂
Phosphate (phosphonooxy ester): -OP(=O)(OR)₂
Phosphite: -OP(OR)₂

Acylamido (acylamino): -NR^{A}C(=O)R^{B}, wherein R^{A} is, for example, hydrogen, a C₁₋₇alkyl group, a C₃₋₂₀ heterocyclyl group, or a C₅₋₂₀ aryl group, preferably hydrogen or a C₁₋₇ alkyl group, and R^{B} is for example, a C₁₋₇ alkyl group, a C₃₋₂₀ heterocyclyl group, or a C₅₋₂₀aryl group, preferably hydrogen or a C₁₋₇ alkyl group. R^{A} and R^{B} may together form a cyclic structure, as in, for example, succinimidyl, maleimidyl, and phthalimidyl:

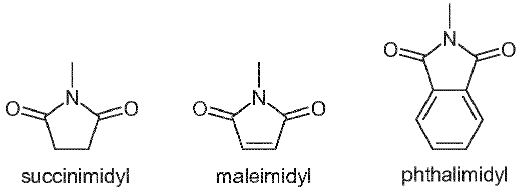

Ureido: -N(R^{C})CONR^{A}R^{B} wherein R^{A} and R^{B} are independently, for example, hydrogen, a C₁₋₇ alkyl group, a C₃₋₂₀ heterocyclyl group, or a C₅₋₂₀ aryl group, preferably H or a C₁₋₇ alkyl group, or, in the case of a "cyclic" group, R^{A} and R^{B}, taken together with the nitrogen atom to which they are attached, form a heterocyclic ring having from 4 to 8 ring atoms, and R^{C} is, for example, hydrogen, a C₁₋₇ alkyl group, a C₃₋₂₀ heterocyclyl group, or a C₅₋₂₀ aryl group, preferably hydrogen or a C₁₋₇alkyl group.

Sulfinamino: -NR^{A}S(=O)R, wherein R^{A} is for example, hydrogen, a C₁₋₇ alkyl group, a C₃₋₂₀ heterocyclyl group, or a C₅₋₂₀ aryl group, preferably H or a C₁₋₇ alkyl group, and R is a sulfinamino substituent, for example, a C₁₋₇ alkyl group, a C₃₋₂₀ heterocyclyl group, or a C₅₋₂₀ aryl group, preferably a C₁₋₇alkyl group.

Phosphoramidite: -OP(OR^{R})-NR^{B}₂, where R^{A} and R^{B} are, for example, H, a C₁₋₇ alkyl group, a C₃₋₂₀ heterocyclyl group, or a C₅₋₂₀ aryl group, preferably H, a C₁₋₇ alkyl group, or a C₅₋₂₀aryl group.

Phosphoramidate: -OP(=O)(OR^{A})-NR^{B}₂, where R^{A} and R^{B} are phosphoramidate substituents, for example, H, a C₁₋₇ alkyl group, a C₃₋₂₀ heterocyclyl group, or a C₅₋₂₀ aryl group, preferably H, a C₁₋₇ alkyl group, or a C₅₋₂₀ aryl group.

### Further definitions

The term "halide" means fluoride, chloride, bromide or iodide.

A "base" in chemistry is a substance that can accept protons or more generally, donate electron pairs. Examples of bases include, among others, sodium hydroxide, potassium hydroxide, calcium hydroxide, alkali metal alkoxides, alkali metal aryloxides, ammonia, pyridine, triethylamine and the like.

The term "metal atom in a metal compound" refers to a metal atom that is part of a coordination compound of said metal with one or more ligands.

The term "ligand" in the present application means an ion, atom, or molecule that binds to a metal. Ligands are usually bases with at least one lone electron pair available for coordination to the metal. The ligand may be monodentate, bidentate, or higher. Suitable ligands include organic compounds containing heteroatoms such as nitrogen, phosphorus, oxygen and sulfur. Examples of such compounds include, but are not limited to, 1,10-phenanthroline and its derivatives, 2,2'-bipyridyl and its derivatives, pyridine and its derivatives, mono-, di- and polydentate phosphines, carbenes, as well as mixed ligands containing two or more different heteroatoms.

The term "cupration" in the present application means a one-step process of direct displacement of a hydrogen atom on a molecule by a copper atom. Therefore, a cuprating reagent is a reagent able to displace in a one-step process a hydrogen atom on a molecule by a copper atom, optionally bearing one or more ligands.

The term "gram-mole" in the present application means the amount of a substance that contains as many molecules as there are atoms in 12 g of the isotope carbon-12 (¹²C).

The term "gram-atom" in the present application means the amount of an element that contains as many of its atoms as there are atoms in 12 g of the isotope carbon-12 (¹²C).

The term "catalyst" in the present application means a substance that increases the rate of a chemical reaction while being used in a molar quantity that is smaller than the molar quantity of the reactants and reagents. For example, platinum metal is a catalyst for the reaction of hydrogen and oxygen to produce water. In another example, palladium is a catalyst for the hydrogenation of olefins. In another example, phosphoric acid is a catalyst for the addition of water to ethylene to give ethanol.

The term "copper (I) source" in the present application means a substance or mixtures of substances containing a copper (I) compound available for a desired transformation involving copper (I). Examples include, among others, copper (I) chloride, copper (I) bromide, copper (I) iodide, copper (I) cyanide, copper (I) oxide, copper (I) trifluoromethanesulfonate, tetrakis(acetonitrile)copper (I) tetrafluoroborate, tetrakis(acetonitrile)copper (I) tetraphenylborate, tetrakis(acetonitrile)copper (I) hexafluorophosphate, tetrakis(acetonitrile)copper (I) trifluoromethanesulfonate, copper (I) sulfide, copper (I) thiocyanate, [Cu[1,3-bis(2,6-diisopropylphenyl)imidazol-2-ylidene]Cl, [Cu[1,3-bis(2,6-diisopropylphenyl)imidazol-2-ylidene]Br, [CuBr(1,10-phenanthroline)]₂, [CuCl(1,10-phenanthroline)]₂, [CuI(1,10-phenanthroline)]₂, copper (I) trifluoroacetate, [CU(PPh₃)₃]Br, [Cu(PPh₃)₃]F, [Cu(PPh₃)₃]Cl, Cu(OCOR²), Cu(SR²), Cu(SR²₂)Br, Cu(SR²₂)Cl, Cu(SR²₂)I, Cu(OSO₂R²), CuOR², wherein R² is selected from alkyl, preferably C₁₋₂₀ alkyl, optionally substituted with one or more aryl, alkoxy and aryloxy and from aryl, preferably C₅₋₇ aryl, optionally substituted with one or more alkyl, aryl, alkoxy and aryloxy and mixtures thereof. In addition, "copper (I) source" in the present application means a substance or mixtures of substances containing copper in any other oxidation state, provided it can be converted to copper in the oxidation state of +1 by means of reduction or oxidation, either chemically or electrochemically.

The term "copper available to form the cuprating reagent" in the present application means the amount of copper atoms present in the reaction mixture that can bind to the oxygen atoms of more than one anion selected from the anions of formula ⁻OR¹, ⁻OR² and mixtures thereof, wherein R¹ and R² are as defined above. In the field of the invention, a ligand can be used to scavenge some copper (I) atoms. Depending on the denticity of the ligand and its amount used, a defined number of copper (I) atoms will be coordinated to that ligand, and thus made unavailable for the process of the invention. In this way, the ratio of copper (I) to the base can be adjusted and controlled to achieve the desired cuprating composition. The "copper available to form the cuprating reagent" refers to those copper (I) atoms that are not coordinated to the ligand and that present the ability to form the compounds of formula **I** in the presence of a base.

A process performed "sequentially" in the present application means a process involving two or more steps, in which a reactant or reagent obtained in one step is stored, either as a composition or as an isolated compound, prior to its use in the next step involving other reagents or reactants.

The expression "optionally substituted with one or more" in the present application means that a group can be substituted with one or more substituents. The substituents can be the same or different and can be placed in any available position.

The term "pentafluoroethylcopper compound" in the present application means a chemical compound comprising a Cu-CF₂CF₃ bond.

The term "atmospheric pressure" in the present application means the force per unit area applied to a surface by the weight of air above that surface in the atmosphere. Atmospheric pressure usually ranges from 0.9 bar to 1.1 bar, preferably the accepted value of atmospheric pressure is 1.013 bar.

A process "performed continuously" in the present application means a flow process that allows for a continuous production of the product by constant feeding of reactants and solvent in the right proportions.

The term "oxidant" in the present application means a chemical compound prompt to accept one or more electrons from another reagent within a RedOx chemical reaction. Examples include, among others, oxygen, perchlorate anions, oxone, percarboxylic acids and peroxides.

The term "electrophile" in the present application means a molecule bearing at least one electron-deficient atom that is prompt to accept one or more electrons from a reagent to allow for the formation of a new chemical bond. Electrophiles useful in the pentafluoroethylation method of the present invention include olefins, alkynes, organic halides and sulfonates, electron-deficient derivatives of Si, Ge, B, P, As, Sb, Bi, S, Se, Te and metal atoms in some metal compounds. Examples of "electrophile" include, among others, aryl halides, heteroaryl halides, alkyl halides, alkynyl halides, alkenyl halides, allyl halides, acyl halides, heterocyclyl halides, aryl sulfonates, heteroaryl sulfonates, alkyl sulfonates, alkynyl sulfonates, heterocyclyl sulfonates, alkenyl sulfonates, allyl sulfonates, boron trichloride and phosphorus trichloride.

The term "organoboron compound" in the present application means a chemical compound containing at least one boron-carbon covalent bond. Examples include, among others, boranes, boronates and boronic acids.

The term "terminal alkyne" in the present application means a chemical compound comprising the group -C≡C-H. The remainder of the molecule may comprising any suitable group, and may in particular be a C₁₋₂₀ alkyl group, a C₅₋₂₀ aryl group or a C₃₋₂₀ hetocyclyl group, optionally subtitued as described above.

### Further embodiments

In some aspects of the invention, the [Cu(OR¹)(OR²)] moiety may be an anion.

### First aspect

In some embodiments, in the cuprating agent comprising the [Cu(OR¹)(OR²)] moiety, R¹ and R² are the same.

In other embodiments, in the cuprating agent comprising the [Cu(OR¹)(OR²)] moiety, R¹ and R² are different.

In further embodiments, the cuprating agent comprising the [Cu(OR¹)(OR²)] moiety is selected from the compounds of formula **II**, a polymer thereof and an oligomer thereof:

MₗSₘCuₓ(OR¹)_{y}(OR²)_{z}X_{q} (**II**)

wherein:
M is selected from the group consisting of lithium, sodium, potassium, rubidium, cesium, calcium, barium and magnesium; and
S is selected from N,N-dimethylformamide (DMF), 1,3-dimethyl-2-imidazolidinone (DMI), N,N-dimethylacetamide (DMAC), 1,3-dimethyl-3,4,5,6-tetrahydro-2(1 H)-pyrimidinone (DMPU), N-methylpyrrolidone (NMP), dimethylsulfoxide (DMSO), tetramethylurea (TMU), and hexamethylphosphoramide (HMPA);
R¹ and R² are as defined above;
X is chloride, bromide or iodide;
l is an integer from 1 to 20;
m is an integer from 0 to 20;
x, y and z are each an integer from 1 to 20;
q is an integer from 0 to 10; and where:
   the sum of I and x is equal to the sum of y, z and q when M is selected from lithium, sodium, potassium, rubidium and cesium; or
   the sum of twice the value of I and x is equal to the sum of y, z and q when M is selected from calcium, magnesium and barium.

### M

In some emobodiments, M is selected from sodium, potassium, rubidium and cesium.

In a further embodiment, M is sodium or potassium.

In an even further embodiment, M is potassium.

### R¹

In some embodiments, R¹ is C₁₋₂₀ alkyl, optionally substituted with one or more C₅₋₇ aryl, C₁₋₂₀ alkoxy or C₅₋₇ aryloxy. In further embodiments, R¹ may be C₁₋₁₀ alkyl, optionally substituted with one or more C₅₋₇ aryl, C₁₋₂₀alkoxy or C₅₋₇ aryloxy, or C₁₋₇ alkyl, optionally substituted with one or more C₅₋₇ aryl, C₁₋₂₀ alkoxy or C₅₋₇ aryloxy.

In some embodiments, R¹ may be unsubstituted C₁₋₂₀ alkyl, and may be unsubstituted C₁₋₁₀ alkyl or unsubstituted C₁₋₇ alkyl.

In an even further embodiment, R¹ is selected from the group consisting of methyl, ethyl, propyl, *iso*-propyl, butyl, *iso*-butyl, *tert*-butyl, pentyl and *neo*-pentyl. Preferably R¹ is methyl or *tert*-butyl; and more preferably R¹ is *tert*-butyl.

In some embodiments, R¹ is C₅₋₂₀ aryl optionally substituted with one or more groups selected from: C₁₋₂₀ alkyl, C₁₋₂₀ alkoxy, C₅₋₂₀ aryl, C₅₋₂₀ aryloxy, halo, nitro and cyano. In further embodiments, R¹ is C₅₋₁₀ aryl optionally substituted with one or more groups selected from: C₁₋₂₀ alkyl, C₁₋₂₀ alkoxy, C₅₋₂₀ aryl, C₅₋₂₀ aryloxy, halo, nitro and cyano, or C₅₋₆ aryl optionally substituted with one or more groups selected from: C₁₋₂₀alkyl, C₁₋₂₀ alkoxy, C₅₋₂₀ aryl, C₅₋₂₀ aryloxy, halo, nitro and cyano.

In some embodiments, R¹ is phenyl optionally substituted with one or more substituents selected from the group consisting of methyl, methyloxy, ethyl, iso-propyl and tert-butyl.

### R²

In some embodiments, R² is C₁₋₂₀ alkyl, optionally substituted with one or more C₅₋₇ aryl, C₁₋₂₀alkoxy or C₅₋₇ aryloxy. In further embodiments, R² may be C₁₋₁₀ alkyl, optionally substituted with one or more C₅₋₇ aryl, C₁₋₂₀alkoxy or C₅₋₇ aryloxy, or C₁₋₇ alkyl, optionally substituted with one or more C₅₋₇ aryl, C₁₋₂₀alkoxy or C₅₋₇ aryloxy.

In some embodiments, R² may be unsubstituted C₁₋₂₀ alkyl, and may be unsubstituted C₁₋₁₀ alkyl or unsubstituted C₁₋₇ alkyl.

In an even further embodiment, R² is selected from the group consisting of methyl, ethyl, propyl, *iso-*propyl, butyl, *iso-*butyl, *tert*-butyl, pentyl and *neo-*pentyl. Preferably R² is methyl or *tert*-butyl; and more preferably R² is *tert*-butyl.

In some embodiments, R² is C₅₋₂₀ aryl optionally substituted with one or more groups selected from: C₁₋₂₀ alkyl, C₁₋₂₀ alkoxy, C₅₋₂₀ aryl, C₅₋₂₀ aryloxy, halo, nitro and cyano. In further embodiments, R² is C₅₋₁₀ aryl optionally substituted with one or more groups selected from: C₁₋₂₀ alkyl, C₁₋₂₀ alkoxy, C₅₋₂₀ aryl, C₅₋₂₀ aryloxy, halo, nitro and cyano, or C₅₋₆ aryl optionally substituted with one or more groups selected from: C₁₋₂₀ alkyl, C₁₋₂₀ alkoxy, C₅₋₂₀ aryl, C₅₋₂₀ aryloxy, halo, nitro and cyano.

In some embodiments, R² is phenyl optionally substituted with one or more substituents selected from the group consisting of methyl, methyloxy, ethyl, iso-propyl and tert-butyl.

### S

In some embodiments, S is selected from N,N-dimethylformamide (DMF), 1,3-dimethyl-2-imidazolidinone (DMI), N-methylpyrrolidone (NMP), hexamethylphosphoramide (HMPA), and N,N-dimethylacetamide (DMAC). In further embodiments, S is selected from N,N-dimethylformamide (DMF), 1,3-dimethyl-2-imidazolidinone (DMI), and N,N-dimethylacetamide (DMAC). In even further embodiments,S is N,N-dimethylformamide (DMF).

### X

In some embodiments, X is chloride.

In some embodiments, X is bromide.

In some embodiments, X is iodide.

### Values

In some embodiments, q is an integer between 0 and 2. In particular, when X is Cl or Br, q may be 0, and when X is I, q may be 2.

In some embodiments, m is an integer between 0 and 10. In further embodiment, m is an integer between 0 and 8. It may be preferred that m is at least 1, thus m may, in some embodiments, be from 1 to 20, 1 to 10 or 1 to 8.

In some embodiments, I is an integer between 1 and 8.

In some embodiments, x, y, and z are each an integer between 1 and 8, or may be an integer between 1 and 6. In some embodiments, x, y and z are different. In other embodiments, x, y and z are the same. In even further embodiments, x, y and z are the same and are an integer between 1 and 6. In some embodiments, the sum of y and z is equal to twice the value of x.

In some embodiments:
I is an integer of between 1 and 8;
m is an integer of between 0 and 8;
x, y and z are the same and are an integer of between 1 and 6;
q is an integer of between 0 and 2.

In some embodiments, the cuprating agent is selected from:
[K₈Cu₆(*tert*-BuO)₁₂(DMF)₈I]I; K(DMF)[Cu(*tert*-BuO)₂]; Na(DMF)₂[Cu(*tert*-BuO)₂]; and polymers or oligomers thereof.

### Reaction with pentafluoroethane

In some embodiments, the solvent for the reaction of the cuprating agent with pentafluroethane is an anhydrous aprotic solvent. In a further embodiment, the solvent for the reaction of the cuprating agent with pentafluroethane is selected from the group consisting of N,N-dimethylformamide (DMF), tetrahydrofurane (THF), 1,4-dioxane, 1,3-dimethyl-2-imidazolidinone (DMI), N,N-dimethylacetamide (DMAC), 1,3-dimethyl-3,4,5,6-tetrahydro-2(1 H)-pyrimidinone (DMPU), N-methylpyrrolidone (NMP), dimethylsulfoxide (DMSO), tetramethylurea (TMU), hexamethylphosphoramide (HMPA) and mixtures thereof. In further embodiments, the solvent is selected from anhydrous N,N-dimethylformamide (DMF), anhydrous N-methylpyrrolidone (NMP) and mixtures thereof.

In some embodiments, the solvent is preferably the same as S in the cuprating reagent; more preferably, the solvent is N,N-dimethylformamide (DMF).

In some embodiments, the amount of pentafluoroethane is from 0.01 to 1000000 gram-moles per each gram-atom of copper (I) in the cuprating agent, preferably from 0.5 to 100 gram-moles per each gram-atom of copper (I) in the cuprating agent, preferably from 0.5 to 10 gram-moles per each gram-atom of copper (I) in the cuprating agent.

In a further embodiment, the amount of pentafluoroethane is 1 gram-mole per each gram-atom of copper (I) in the cuprating agent.

In some embodiments, the reaction of the cuprating agent and pentafluroethane is performed by contacting the reactants at stirring for from 1 second to 48 hours, preferably for from 1 minute to 24 hours.

In some embodiments, the reaction of the cuprating agent and pentafluroethane is carried out at a temperature from -30°C to 100°C, preferably from -30°C to 80°C, more preferably from -10°C to 50°C, even more preferably from 15°C to 30°C, and still more preferably at room temperature.

In some embodiments, the reaction of the cuprating agent and pentafluroethane is performed under a pressure from 0.1 to 50 bar, preferably under atmospheric pressure.

In some embodiments, the reaction of the cuprating agent and pentafluroethane is performed at a concentration of cuprating agent comprised of from 0.01 to 20 M, more preferably from 0.05 to 4 M and even more preferably from 0.3 to 0.7 M.

### Treatment of pentafluoroethylating composition with acid

In some embodiments of the first aspect of the invention, the invention provides a process to obtain a pentafluoroethylating composition, further comprising the step of treating the pentafluoroethylating composition obtained by reacting the cuprating agent with pentafluoroethane with an acid. The acid may be selected from the group consisting of hydrogen fluoride, hydrogen chloride, acetic acid, trifluoroacetic acid, formic acid, hydrogen fluoride pyridine, mixtures of compounds of formula NR³R⁴R⁵ with hydrogen fluoride, melamine/HF, poly[4-vinylpyridinium poly(hydrogen fluoride)], mixtures thereof and B(OR⁶)₃; wherein R³, R⁴, R⁵ and R⁶ are independently unsubstituted C₁₋₂₀ alkyl.

Preferably the acid is selected from the group consisting of hydrogen fluoride, hydrogen fluoride pyridine, hydrogen chloride, acetic acid, triethylamine trihydrofluoride, melamine/HF, poly[4-vinylpyridinium poly(hydrogen fluoride)], mixtures thereof and B(OR⁶)₃, wherein R⁶ is C₁₋₂₀ alkyl; even more preferably the acid is selected from the group consisting of hydrogen fluoride, hydrogen fluoride pyridine, hydrogen chloride, acetic acid, triethylamine trihydrofluoride, mixtures thereof and B(OR⁶)₃, wherein R⁶ is C₁₋₂₀ alkyl. Most preferably, the acid is selected from the group consisting of hydrogen fluoride, hydrogen fluoride pyridine, hydrogen chloride, acetic acid, triethylamine trihydrofluoride, mixtures thereof and B(OCH₃)₃. In particular embodiments, the acid is triethylamine trihydrofluoride. In other particular embodiments, the acid is hydrogen fluoride pyridine. In other particular embodiments, the acid is hydrogen chloride. In other particular embodiments, the acid is acetic acid.

Where the pentafluoroethylating composition is treated with an acid, the acid is preferably used in the amount of from 0.1 to 5 gram-atom of protons per each gram-atom of copper (I) of the pentafluoroethylating composition; more preferably the acid is used in the amount of from 1 to 3 gram-atom of protons per each gram-atom of copper (I) of the pentafluoroethylating composition.

In another embodiment, the invention provides a process to obtain a pentafluoroethylating composition, wherein the pentafluoroethylating composition is treated with an acid as defined above; and wherein the acid is added neat.

In another embodiment, the invention provides a process to obtain a pentafluoroethylating composition, wherein the pentafluoroethylating composition obtained is treated with an acid as defined above; and wherein the acid is added in solution. Preferably the solvent is selected from the group consisting of N,N-dimethylformamide (DMF), 1,3-dimethyl-2-imidazolidinone (DMI), N,N-dimethylacetamide (DMAC), 1,4-dioxane, tetrahydrofuran, 1,3-dimethyl-3,4,5,6-tetrahydro-2(1H)-pyrimidinone (DMPU), N-methylpyrrolidone (NMP), dimethylsulfoxide (DMSO), tetramethylurea (TMU), hexamethylphosphoramide (HMPA) and mixtures thereof. More preferably the solvent is selected from anhydrous N,N-dimethylformamide (DMF), anhydrous N-methylpyrrolidone (NMP) and mixtures thereof. Most preferably the acid is added in solution of the solvent used in the reaction of the cuprating agent with pentafluroethane.

In another embodiment, the invention provides a process to obtain a pentafluoroethylating composition, wherein after the treatment of the pentafluoroethylating composition with an acid, the alcohol produced is removed from the mixture under vacuum, via adsorption or via chemisorption.

### Making the cuprating agent

In some embodiments of the first aspect, the method comprises the initial step of contacting a copper (I) source and a base in the presence of a solvent to form a cuprating reagent, where the base is a compound of formula **I** or a mixture of compounds of formula **I** as defined above.

In some of these embodiments, the base is selected from potassium tert-butoxide, sodium *tert*-butoxide, potassium methoxide and mixtures thereof. In further embodiments, the base is selected from potassium *tert*-butoxide, sodium *tert*-butoxide and mixtures thereof. In even further embodiments, the base is selected from potassium *tert*-butoxide, potassium methoxide and mixtures thereof. Preferably, the base is potassium *tert-*butoxide.

In other embodiments, M and R¹ in the base of formula **I** are as described above.

In some embodiments, the initial step of producing the cuprating agent and the subsequent step of reacting the cuprating agent with pentafluroethane are performed sequentially. In further of these embodiments, the cuprating agent is stored prior to its use in reacting with pentafluoroethane, preferably for from 0.5 minutes to 30 days. In other further embodiments, the cuprating reagent is isolated and stored prior to its use, and preferably protected from oxygen and moisture, prior to its use in reacting with pentafluoroethance.

In other embodiments, the intial step of producing the cuprating agent and the reaction of the cuprating agent with pentafluoroethane are performed by mixing together the copper (I) source, the base of formula **II**, the solvent and pentafluoroethane.

In some embodiments, the solvent in the initial step of producing the cuprating agent is a polar aprotic solvent. In further embodiments, the solvent is selected from the group consisting of: N,N-dimethylformamide (DMF), 1,3-dimethyl-2-imidazolidinone (DMI), N-dimethylacetamide (DMAC), 1,3-dimethyl-3,4,5,6-tetrahydro-2(1H)-pyrimidinone (DMPU), N-methylpyrrolidone (NMP), dimethylsulfoxide (DMSO), tetramethylurea (TMU), hexamethylphosphoramide (HMPA), and mixtures thereof.

In some embodiments, the solvent in the initial step of producing the cuprating agent is anhydrous. In further embodiments, the solvent is selected from anhydrous N,N-dimethylformamide (DMF), anhydrous 1,3-dimethyl-2-imidazolidinone (DMI), anhydrous N-dimethylacetamide (DMAC), anhydrous 1,3-dimethyl-3,4,5,6-tetrahydro-2(1H)-pyrimidinone (DMPU), anhydrous N-methylpyrrolidone (NMP), anhydrous dimethylsulfoxide (DMSO), anhydrous tetramethylurea (TMU), anhydrous hexamethylphosphoramide (HMPA), and mixtures thereof. Preferably the solvent is selected from anhydrous N,N-dimethylformamide (DMF), anhydrous N-methylpyrrolidone (NMP) and mixtures thereof.

The solvent in the intial step of producing the cuprating agent may be the same as S in formula **II**.

In some embodiments, the copper (I) source is generated in situ from a copper (II) or a copper (III) compound and a reducing agent.

In some embodiments, the copper (I) source is generated in situ from metallic copper and an oxidizing agent.

In some embodiments, the copper (I) source is selected from copper (I) chloride, copper (I) bromide, copper (I) iodide, copper (I) cyanide, copper (I) oxide, copper (I) trifluoromethanesulfonate, copper (I) thiocyanate, [Cu[1,3-bis(2,6-diisopropylphenyl)imidazol-2-ylidene]Cl, [Cu[1,3-bis(2,6-diisopropylphenyl)imidazol-2-ylidene]Br, [CuBr(1,10-phenanthroline)]₂, [CuCl(1,10-phenanthroline)]₂, [CuI(1,10-phenanthroline)]₂, copper (I) trifluoroacetate, Cu(OR³), Cu(OCOR³), Cu(SR³), Cu(SR3₂)Br, Cu(SR³₂)Cl, Cu(SR³₂)I, Cu(OSO₂R³) and mixtures thereof, wherein R³ is C₁₋₂₀ alkyl or C₅₋₂₀ aryl, wherein C₁₋₂₀ alkyl is optionally substituted with one or more groups selected from: C₅₋₂₀ aryl, C₁₋₂₀alkoxy and C₅₋₂₀ aryloxy, and wherein C₅₋₂₀ aryl is optionally substituted with one or more groups selected from: C₁₋₂₀ alkyl, C₁₋₂₀ alkoxy, C₅₋₂₀ aryl, C₅₋₂₀ aryloxy, halo, nitro and cyano. In further embodiments, the copper (I) source is selected from copper (I) chloride, copper (I) bromide, copper (I) iodide, copper (I) cyanide, copper (I) oxide, copper (I) trifluoromethanesulfonate, copper (I) thiocyanate, [Cu[1,3-bis(2,6-diisopropylphenyl)imidazol-2-ylidene]Cl, [Cu[1,3-bis(2,6-diisopropylphenyl)imidazol-2-ylidene]Br, [CuBr(1,10-phenanthroline)]₂, [CuCl(1,10-phenanthroline)]₂, [Cul(1,10-phenanthroline)]₂, copper (I) trifluoroacetate, Cu(OCOR³), Cu(SR³), Cu(SR³₂)Br, Cu(SR³₂)Cl, Cu(SR³₂)I, Cu(OSO₂R³) and mixtures thereof, wherein R³ is as defined above. In even further embodiments, the copper (I) source is selected from CuOR³ and mixtures thereof, wherein R³ is as defined above.

In particular embodiments, the copper (I) source is selected from copper (I) chloride, copper (I) bromide, copper iodide, copper (I) acetate, copper (I) trifluoromethanesulfonate, and mixtures thereof. In more particular embodiments, the copper (I) source is selected from copper (I) chloride, copper (I) bromide, copper iodide and mixtures thereof, preferably the copper (I) source is copper (I) chloride.

In some embodiments, the amount of base is from 1.1 gram-moles to 4 gram-moles per each gram-atom of copper (I) in the copper (I) source, preferably from 1.2 gram-moles to 2.5 gram-moles per each gram-atom of copper (I) in the copper (I) source, and more preferably is 2 gram-moles per each gram-atom of copper (I) in the copper (I) source.

In some embodiments, the amount of base is from 1.1 gram-moles to 4 gram-moles per each gram-atom of copper (I) in the copper available to form the cuprating reagent, preferably from 1.2 gram-moles to 2.5 gram-moles per each gram-atom of copper (I) in the copper available to form the cuprating reagent, and more preferably is 2 gram-moles per each gram-atom of copper (I) in the copper available to form the cuprating reagent.

In preferred embodiments, the copper (I) source is selected from copper (I) chloride, copper (I) bromide, copper (I) iodide, copper (I) cyanide, copper (I) oxide, copper (I) trifluoromethanesulfonate, copper (I) thiocyanate, [Cu[1,3-bis(2,6-diisopropylphenyl)imidazol-2-ylidene]Cl, [Cu[1,3-bis(2,6-diisopropylphenyl)imidazol-2-ylidene]Br, [CuBr(1,10-phenanthroline)]₂, [CuCl(1,10-phenanthroline)]₂, [Cul(1,10-phenanthroline)]₂, copper (I) trifluoroacetate, Cu(OCOR³), Cu(SR³), Cu(SR³₂)Br, Cu(SR³₂)Cl, Cu(SR³₂)I, Cu(OSO₂R³) and mixtures thereof, wherein R³ is C₁₋₂₀ alkyl or C₅₋₂₀ aryl, wherein C₁₋₂₀alkyl is optionally substituted with one or more groups selected from: C₅₋₂₀ aryl, C₁₋₂₀alkoxy and C₅₋₂₀ aryloxy, and wherein C₅₋₂₀ aryl is optionally substituted with one or more groups selected from: C₁₋₂₀ alkyl, C₁₋₂₀alkoxy, C₅₋₂₀ aryl, C₅₋₂₀ aryloxy, halo, nitro and cyano; and
the amount of base is 2 gram-moles per each gram-atom of copper (I) in the copper available to form the cuprating reagent.

In some embodiments, the amount of base is from 0.1 gram-moles to 3 gram-moles per each gram-atom of copper (I) in the copper (I) source, preferably from 1 gram-mole to 1.5 gram-moles per each gram-atom of copper (I) in the copper (I) source, and more preferably is 1 gram-mole per each gram-atom of copper (I) in the copper (I) source.

In some embodiments, the amount of base is from 0.1 gram-moles to 3 gram-moles per each gram-atom of copper (I) in the copper available to form the cuprating reagent, preferably from 1 gram-mole to 1.5 gram-moles per each gram-atom of copper (I) in the copper available to form the cuprating reagent, and more preferably is 1 gram-mole per each gram-atom of copper (I) in the copper available to form the cuprating reagent.

In other preferred embodiments, the copper (I) source is selected from CuOR³ and mixtures thereof, wherein R³ is as defined above; and the amount of base is 1 gram-mole per each gram-atom of copper (I) in the copper available to form the cuprating reagent.

In some embodiments, the step of making the cuprating reagent is carried out at a temperature from -30°C to 100°C, preferably from -30°C to 80°C, more preferably from -10°C to 50°C, even more preferably from 15°C to 30°C, and still more preferably at room temperature.

In some embodiments, the step of making the cuprating reagent is carried out by stirring for from 1 minute to 18 days, preferably stirring for from 1 minute to 48 hours, and more preferably stirring for from 0.1 hours to 10 hours.

In some embodiments, the step of making the cuprating reagent is performed by mixing the copper (I) source, the base and the solvent in any order and stirring for from 1 minute to 10 hours.

In some embodiments, the step of making the cuprating reagent is performed under a pressure from 0.1 to 50 bar, preferably under atmospheric pressure.

### Second aspect

In some embodiments of the second aspect of the present invention, there is provided a pentafluroethylating composition which comprises the moiety of formula [(C₂F₅)Cu(OR¹)], wherein R¹ is as defined in the first aspect.

In some embodiments of the second aspect of the present invention, there is provided a pentafluroethylating composition comprising a compound of formula **III** or a polymer or an oligomer thereof:

M_{l'}S_{m'}Cu_{x'}(OR¹)_{y'}(C₂F₅)_{z'}X_{q'} (**III**)

wherein:
Cu is in the oxidation state +1;
M, S, R¹ and X are as defined in the first aspect;
l' is an integer from 1 to 20;
m' is an integer from 0 to 20;
x', y' and z' are each an integer from 1 to 20;
q' is an integer from 0 to 10; and where:
the sum of I' and x' is equal to the sum of y', z' and q' when M is selected from lithium, sodium, potassium, rubidium and cesium; or,
the sum of twice the value of I' and x' is equal to the sum of y', z' and q' when M is selected from calcium, magnesium and barium.

The embodiments and preferences for M, S, R¹ and X expressed for the first aspect of the invention apply to the second aspect of the invention also.

In some embodiments, q' is an integer between 0 and 2. In particular, when X is Cl or Br, q' may be 0, and when X is I, q' may be 0 or 2, and it may be 0.

In some embodiments, m' is an integer between 0 and 10. In further embodiment, m' is an integer between 0 and 8 or even 0 to 5. It may be preferred that m' is at least 1, thus m' may, in some embodiments, be from 1 to 20, 1 to 10 or 1 to 8.

In some embodiments, I' is an integer between 1 and 8. In further embodiments, I' is 1.

In some embodiments, x', y' and z' are different. In other embodiments, x', y' and z' are the same. In some embodiments, the sum of y' and z' is equal to twice the value of x'. In some embodiments, x', y' and z' are each an integer between 1 and 8, or may be an integer between 1 and 6. In further embodiments, x', y' and z' are 1. In some embodiments, the sum of y' and z' is equal to twice the value of x'.

In some embodiments:
q' is 0;
m' is an integer from 0 to 5;
l' is 1; and
x', y' and z' are 1.

In some embodiments, the pentafluoroethylating agent is K(DMF)₂Cu(O-tert-Bu)(C₂F₅).

### Third aspect

In some embodiments, the invention provides the use of the pentafluoroethylating composition in the transfer of a pentafluoroethyl group to the carbon atom of an organoboron compound wherein the organoboron compound is a boronic acid derivative. Preferably the use of the pentafluoroethylating composition in the transfer of a pentafluoroethyl group to the carbon atom of a boronic acid further comprises an oxidant; and more preferably the oxidant comprises oxygen, and even more preferably the oxidant is air.

The organoboron compound may be of formula (**IV**):

R^{BN}-B(OH)₂ (**IV**)

where R^{BN} is selected from optionally susbtitued C₅₋₂₀ aryl, C₁₋₂₀ alkyl and C₃₋₂₀ heterocyclyl. In further embodiments, R^{BN} is C₅₋₂₀ aryl. The optional substituents are as described above.

The boronic acid group may be replaced by boronate esters, such as pinacol esters or trimethylene glycol esters.

In some embodiments, the invention provides the use of the pentafluoroethylating composition defined above in the transfer of a pentafluoroethyl group to an electrophile wherein the electrophile is selected from: C₅₋₂₀ aryl halides, C₃₋₂₀ heterocyclyl halides, C₁₋₂₀ alkyl halides, acyl halides, C₅₋₂₀ aryl sulfonates, C₃₋₂₀ heterocyclyl sulfonates, C₁₋₂₀ alkyl sulfonates, electron-deficient derivatives of Si, Ge, B, P, As, Sb, Bi, S, Se, Te and a metal atom in a metal compound. Preferably the electrophile is selected from the group consisting of: C₅₋₂₀ aryl halides, C₃₋₂₀ heterocyclyl halides, C₁₋₂₀ alkyl halides, acyl halides, C₅₋₂₀ aryl sulfonates, C₃₋₂₀ heterocyclyl sulfonates and C₁₋₂₀ alkyl sulfonates. More preferably the electrophile is selected from the group consisting of: C₅₋₂₀ aryl chlorides, C₅₋₂₀ aryl bromides and C₅₋₂₀ aryl iodides.
In some embodiments, the invention provides the use of the pentafluoroethylating composition defined above wherein the electrophile is selected from an electron-deficient derivative of Si, Ge, B, P, As, Sb, Bi, S, Se and Te, and more preferably selected from an electron-deficient derivative of Si and B.

In some embodiments, the invention relates to the use of the pentafluoroethylating composition defined above wherein the electrophile is a metal atom in a metal compound. Preferably the metal is selected from Pd, Pt, Ni, Zn, Cd, Co, Fe, Ru, Rh and Ir.

In some embodiments, the present invention relates to the use of the pentafluoroethylating composition defined above in the transfer of a pentafluoroethyl group to a terminal alkyne. The terminal alkyne may be of formula (**V**):

R^{AL}-C≡C-H (**V**)

where R^{AL} is selected from optionally susbtitued C₅₋₂₀ aryl, C₁₋₂₀ alkyl and C₃₋₂₀ heterocyclyl. The optional substituents are as described above.

In another embodiment, the invention provides the use of the pentafluoroethylating composition in the transfer of a pentafluoroethyl group to an electrophile, in the presence of a catalyst, wherein the catalyst is selected from metal compounds of Pd, Ni, Pt, Co, Fe, Ru, Rh, Ir and Mn.

In some embodiments, the pentafluoroethylating composition is used at a temperature from -30°C to 180°C, preferably from 20°C to 90°C.

In some embodiments, the pentafluoroethylating composition is reacted for from 1 minute to 30 days, preferably stirring for from 1 minute to 72 hours.

In some embodiments, the pentafluoroethylating composition is used under pressure from 0.1 to 50 bar, and more preferably under atmospheric pressure.

### Fourth aspect

In some embodiments, this aspect of the invention provides the use of the pentafluoroethylating composition of the second aspect of the invention in the preparation of pentafluoroethyl copper complexes with one or more ligands, each of which comprising one or more atoms of group V (group 15).

This aspect of the invention may be achieved by reacting the pentafluoroethylating composition of the second aspect of the invention with the one or more ligands. The aspect may also further comprise the step of isolation of the resulting complex.

In some embodiments, the complexes have one ligand. In other embodiments, the complexes have two ligands. In further embodiments, the complexes have three ligands. In the above embodiments, the atoms of group V may be selected from P and N. In a particular embodiment, the atoms of group V are P. In another particular embodiment, the atoms of group V are N.

In further embodiments of this aspect, the one or more ligands may be a phosphine or a phosphite. In a particular embodiment, the one or more ligands may be a phosphine. More particularly, the one or more ligands may be selected from triphenylphosphine, tri(n-butyl)phosphine, tri(cyclohexylphosphine), tri(p-tolyl)phosphine and mixtures thereof. Most particularly, the one or more ligands is triphenylphosphine.

In other further embodiments of this aspect, the one or more ligand is a heteroaromatic ring containing nitrogen, which heteroaromatic ring may be the parent of the C₅₋₂₀ heteroayl groups described above. In some of these embodiments, the ligand may be selected from the group consisting of: pyrrole, pyridine, oxazole, isoxazole, isoxazine, imidazole, pyrazole, pyridazine, pyrimidine, triazole, indole, benzimidazole, indazole, quinoline, isoquinoline, quinolizine, benzoxazine, benzodiazine, 2,2'-bipyridine, quinoxaline, quinazoline, phthalazine, naphthyridine, pteridine, acridine, phenazine and 1,10-phenanthroline. More particularly, the one or more ligands may be selected from the group consisting of pyridine, 2,2'-bipyridyl, 1,10-phenanthroline, quinoline, and isoquinoline. Most particularly, the one or more ligands is selected from the group consisting of pyridine, 2,2'-bipyridyl and 1,10-phenanthroline.

In other further embodiments of this aspect, the one or more ligand comprises a carbene. In particular, the ligand is a carbene deriving from an imidazolium salt. More particularly, the ligand may be an N-heterocyclic carbene. Even more particularly, the ligand is selected from 1,3-bis(2,6-diisopropylphenyl)imidazol-2-ylidene, 1,3-bis(1-adamantyl)imidazol-2-ylidene, 1,3-bis(tert-butyl)imidazol-2-ylidene, 1,3-diisopropylimidazol-2-ylidene, and 1,3-bis(2,4,6-trimethylphenyl)imidazol-2-ylidene.

In certain embodiments, this aspect of the present invention relates to the use of the pentafluoroethylating composition of the second aspect of the invention in the preparation of a pentafluoroethyl copper complex comprising the moiety [Cu(C₂F₅)X] wherein X is selected from (PR₃³)₂, (PR³₃)₃, L and (PR³₃)(L), wherein R³ is selected from the group consisting of: phenyl, *n*-butyl, cyclohexyl and *p*-tolyl; and L is a bidentate ligand containing at least two atoms of the group V, as described above.

In a particular embodiment, L is selected from 2,2'-bipyridine and 1,10-phenanthroline.

In another particular embodiment, the present invention relates to the use of the pentafluoroethylating composition of the second aspect of the invention in the preparation of a pentafluoroethyl copper complex comprising the moiety [Cu(C₂F₅)(PPh₃)₂]. In certain further embodiments, the present invention relates to the use of the pentafluoroethylating composition of the second aspect of the invention in the preparation of a pentafluoroethyl copper complex that is selected from [Cu(C₂F₅)(PPh₃)₂], [Cu(C₂F₅)(PPh₃)₃] and mixtures thereof.

### Fifth aspect

The embodiments of this aspect may be the complexes made in the embodiments of the fourth aspect of the invention.

In certain aspects, the complex may comprise the moiety [Cu(C₂F₅)X] wherein X is as defined in the fourth aspect of the invention.

In particular embodiments, X is selected from (PR³₃)₂ and (PR³₃)₃ wherein R³ is selected from phenyl, *n*-butyl, cyclohexyl and *p*-tolyl. More particularly, R³ is phenyl.

In particular embodiments, L is 2,2'-bipyridine or 1,10-phenanthroline. More particularly, L is 1,10-phenanthroline.

### General statements

In some of the processes described in the third aspect of the present invention it may be necessary or advisable to protect the reactive or labile groups by conventional protecting groups. Both the nature of these protecting groups and the procedures for their introduction or removal are well known in the art (see for example Wuts, P.G.M and Greene, T.W., "Greene's Protective Groups in Organic Synthesis", John Wiley & Sons, 4th edition, 2007, which is incorporated herein by reference). Whenever a protecting group is present, a later deprotection step will be required, which can be performed under standard conditions in organic synthesis, such as those described in the above-mentioned reference.

Unless otherwise stated, in the methods described below the meanings of the different substituents are the meanings described above.

As it will be obvious to those skilled in the art, interconversion reactions can be carried out upon the compounds of the invention as well as upon any suitable synthesis intermediates thereof.

Throughout the description and claims the word "comprises" and variations of the word, are not intended to exclude other technical features, additives, components or steps. Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

In accordance with this invention, it has been discovered that reagents comprising a copper (I) alkoxide adduct in an organic solvent are useful for reacting with pentafluoroethane to directly produce synthetically useful pentafluoroethyl copper reagents having the CuC₂F₅ moiety. These pentafluoroethyl copper compounds are useful in a variety of pentafluoroethylation reaction to make valuable products.

A number of methods are available for the introduction of the smallest perfluoroalkyl group, CF₃, into organic substrates. Pentafluoroethylation reactions are vastly less developed, despite the fact that in certain cases C₂F₅ derivatives exhibit properties that are superior to those of their CF₃ counterparts [Andrzejewska 2002, Johansson 2003]. Carbanionic sources such as C₂F₅Li have been developed for pentafluoroethylation of carbonyl compounds. The carbanion methodology, however, is not applicable to pentafluoroethylation of aromatic and other organic halides as well as a number of important substrates of different types, such as boronic acids, acetylenes, and inorganic and organometallic compounds. Furthermore, pentafluoroethylated aromatic compounds cannot be made by the Swarts-type process that is used to manufacture trifluoromethylated aromatic compounds. Currently available synthetic routes to aromatic C₂F₅ derivatives are not only scarce, but also suffer from a narrow substrate scope and limited functional group tolerance. The decarboxylative pentafuoroethylation of aryl iodides with CuI/C₂F₅CO₂M (M = Na, K, Me) occurs at 150-180°C, too high a temperature for a number of functional groups to survive. Highly costly and available only in limited quantities C₂F₅SiMe₃ cannot be used with such functionalities as aldehydes, ketones, carboxylic acids, esters, amides, acid chlorides, etc. Pentafluoroethylation of unactivated bromoarenes such as bromobenzene is unknown. By "unactivated" bromoarenes are meant bromobenzenes not containing electron-withdrawing substituents on the aromatic ring.

In attempts to extend the cupration reaction of fluoroform, CHF₃, [Zanardi 2011] to other H-poly- and H-perfluoroalkyl substrates (R_{f}H), a series of experiments were performed, in which the cuprating reagent was contacted with one of the following compounds: CF₃(CF₂)₃H, CF₃(CF₂)₅H, CF₃(CF₂)₇H, HCF₂(CF₂)₄CF₂H, and (CF₃)₂CFH. Although the conditions were identical to the ones employed in the reaction of CHF₃ (Zanardi), not a single of these experiments produced selectively R_{f}Cu but rather gave a complex mixture of organofluorine products (¹⁹F NMR) and KF and/or KHF₂. The formation of inorganic fluoride/bifluoride (KF/KHF₂) indicated that the higher R_{f}H underwent HF elimination rather than selective cupration.

Totally unexpectedly, when pentafluoroethane, C₂F₅H, was used under the same conditions, smooth and clean cupration occurred to give a C₂F₅Cu derivative in nearly quantitative yield. The abrupt change in reaction pathways for the CF₃(CF₂)ₙH/[K(DMF)][(*t*-BuO)₂Cu] system when going from n = 1 (cupration) to n > 1 (HF elimination) was non-obvious. There have been no literature reports on the preparation of CuC₂F₅ compounds directly from C₂F₅H.

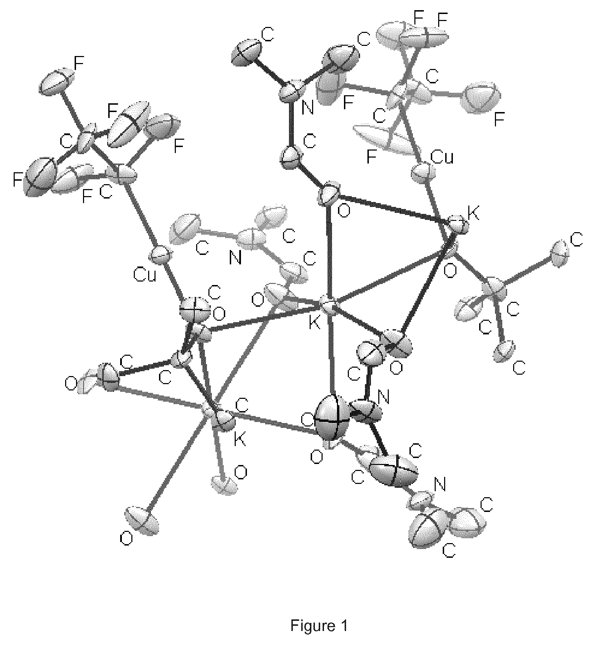

Adding C₂F₅H to [K(DMF)][(*t-*BuO)₂Cu] (prepared as described by Zanardi), pre-isolated or generated in situ from CuCl and *t*-BuOK (1:2), in DMF at room temperature resulted in instantaneous reaction. ¹⁹F NMR analysis of the resultant solution indicated the formation of "CuC₂F₅" (ca. 95%) along with traces (<1%) of [Cu(C₂F₅)₂]⁻. This CuC₂F₅ product appeared to be much more robust than its CuCF₃ analogue prepared in a similar manner from CHF₃. This difference in stability is accounted for by less facile α-F-elimination from higher R_{f}M complexes as compared to their CF₃M congeners. For instance, under conditions where C₂F₅Li is stable, CF₃Li decomposes to LiF and CF₂. The enhanced stability of the C₂F₅H cupration product allowed for its isolation and single-crystal X-ray diffraction study. The structure of this compound, [K(DMF)₂][(C₂F₅)Cu(OBu*^{t}*)], is shown in Figure 1. It is likely that the less stable and consequently non-isolable primary product of the cupration of fluoroform (Zanardi) has a similar structure.

It is preferred to use anhydrous aprotic solvents in the present invention because water and protic solvents hydrolyze, solvolyze and otherwise decompose the cuprating reagent. However, small quantities of water in the system can be tolerated, for example wherein the solvent contains water in the amounts (v/v) 0.005%, 0.01%, 0.05%, 0.1%, and 0.5%. The cuprating reagent is are oxygen-sensitive and therefore are preferably prepared and handled in an inert atmosphere of, for example, nitrogen or argon. The pentafluoroethylating composition may also be oxygen-sensitive and therefore the same precuations may be applied.

The cuprating reagent is stable at room temperature and can be used to cuprate pentafluroethane. To obtain the pentafluoroethyl copper compound from the reagent and pentafluoroethane, the two are contacted. Pentafluoroethane is a gas (b. p. = ca. -48.5°C) that is easily soluble in organic solvents. Pentafluoroethane can be bubbled through the reagent solution or introduced in the headspace. Pentafluoroethane can be added as a solution in an organic solvent that does not decompose the reagent. Alternatively, the cuprating reagent solution can be added to pentafluoroethane either as a gas or dissolved in an organic solvent. The cupration is most conveniently carried out at room temperature and atmospheric pressure, although lower and higher temperatures and pressures from 0.1 to 50 atm maybe applied if necessary. Depending on conditions used, the cupration process can go to completion after as little as a few seconds to several hours. The cupration reaction can be conveniently monitored by ¹⁹F NMR spectroscopy because Cu-C₂F₅ compounds display signals in the characteristic range of -80 to -110 ppm.

The pentafluoroethane reagents appear to be more stable at room temperature, than the trifluoromethyl copper reagents described in WO 2012/113726. Stability has also been demonstrated at 80°C for at least two hours. Treating the pentafluoroethane reagents with acid appears to render the reagents more active (see examples 11, 12 13).

The produced pentafluoroethyl copper reagents can be used to introduce the pentafluoroethyl group into organic and inorganic compounds in manners that are similar to those already known for trifluoromethyl copper reagents obtained by means other than the direct cupration of pentafluoroethane. Both non-stabilized and stabilized solutions can be used, although higher yields may be obtained with the stabilized Cu₂CF₅ reagents. Thus, the ability of the CuC₂F₅ to pentafluoroethylate various substrates was explored. First, aryl iodides were tested. For initial studies, 4-fluoroiodobenzene was chosen as a substrate in order to extract more information from monitoring the reactions by ¹⁹F NMR. No reaction of the CuC₂F₅ generated from CuCl/*t*-BuOK (1:2) and C₂F₅H in DMF was observed on addition of 10 equiv of 4-IC₆H₄F at room temperature, and only small amounts of 4-C₂F₅C₆H₄F were produced after 3 hours at 50°C. After approximately 10 hours at 80°C, however, full conversion of the CuC₂F₅ was reached to 4-C₂F₅C₆H₄F (¹⁹F NMR: δ = -84.4 ppm, 3F; -106.8 ppm, 1 F; -113.2 ppm, 2F) and 4-*t*-BuOC₆H₄F (¹⁹F NMR: δ = -120.3 ppm) in a 1:1.2 ratio. Remarkably, no detectable side decomposition of the CuC₂F₅ took place. These results indicated that (i) the CuC₂F₅ is less reactive toward aryl halides, yet much more thermally stable than its CF₃ analogue and (ii) like fluoroform-derived CuCF₃ prior to the stabilization, the CuC₂F₅ both fluoroalkylates and *tert-*butoxylates the substrate.

The t-butoxylation side-reaction was efficiently suppressed by adding Et₃N·3HF (TREAT HF) or Py·nHF to CuC₂F₅ in DMF prior to use in pentafuloroethylation reactions. This technique was adopted from the previous work with fluoroform-derived CuCF₃. Interestingly, it was found that not only HF sources but also other acids such as AcOH or HCl in dioxane could be used with the CuC₂F₅ to eliminate the undesired *t*-butoxylation.

Optimization experiments indicated that adding 1.6 equiv of HF in the form of TREAT HF to a solution of CuC₂F₅ produced the most efficient reagent. The latter was found to pentafuoroethylate iodoarenes in 90-99% yield at 23-50°C, as described in more detail in the Examples. A slight 20% excess of CuC₂F₅ was sufficient to achieve full conversion of the starting Arl substrates. Electron-withdrawing and electron-donating substituents in the *o*-, *m*-, and *p*-positions were easily tolerated, including simple alkyls, MeO, CHO, Cl, Br, CF₃, CO₂Et, CH₃CO, NO₂, and CONH₂. 1-Iodonaphthalene and 2-iodopyridine underwent smooth pentafluoroethylation to give the corresponding products in 97% and 95% yield, respectively.
Much more cost-attractive and readily available aryl bromides are significantly less reactive than iodoarenes. There have been no reports in the open or patent literature of general methods for efficient pentafluoroalkylation of unactivated bromoarenes such as bromobenzene. As described in the Examples, the CuC₂F₅ reagent pentafluoroethylates various bromoarenes at 80-90°C with high selectivity in 80-99% yield. Even particularly challenging bromobenzene, *o*- and *p*-bromoanisoles, and *m*-bromotoluene were smoothly converted to the corresponding C₂F₅ derivatives in over 90% yield. 2-(pentafluoroethyl)naphthalene was produced in the reaction in 95% and isolated in 82% yield.
The CuC₂F₅ was found to be useful for pentafluoroethylation of not only aryl halides but also a variety of other substrates (Scheme 1), including aryl boronic acids and terminal acetylenes under oxidative conditions (in air), benzylic and vinylic bromides, and metal complexes. Treatment of [(tmeda)Pd(Ph)I] with our CuC₂F₅ reagent gave [(tmeda)Pd(C₂F₅)(Ph)] (**6**) that was isolated in 90% yield and fully characterized, including by single-crystal X-ray diffraction. Palladium complexes bearing a σ-C₂F₅ ligand are extremely rare because of the lack of general methods for Pd-C₂F₅ bond formation. Also, the CuC₂F₅ can be used to prepare other Cu complexes bearing a σ-C₂F₅ ligands. For example, treatment of the C₂F₅H-derived CuC₂F₅ with PPh₃ produced a new complex, [(Ph₃P)₂CuC₂F₅], that was isolated and structurally characterized by X-ray analysis. Experimental details of the above-described experiments are provided in the Examples section.

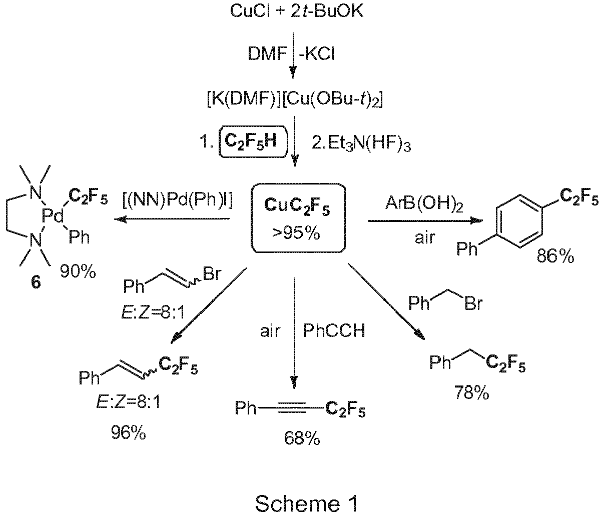

In certain cases the final pentafluoroethylation step may be conveniently combined with the previous step, which is the formation of the pentafluoroethyl reagent from pentafluoroethane. In certain other cases, pentafluoroethylation reactions with the pentafluoroethyl copper reagents made by this invention can be beneficially conducted in the presence of a metal catalyst such as a palladium compound.

The present invention is beneficial in that it allows for the preparation of valuable pentafluoroethyl copper reagents in high yield, directly, selectively and efficiently from pentafluoroethane. Pentafluoroethane is the most attractive pentafluoroethyl source, being readily available in large industrial quantities and inexpensive. The use of pentafluoroethane as described above is highly economical because the process employs relatively inexpensive chemicals and materials and can be conveniently conducted at room temperature and atmospheric pressure. In contrast, the known indirect processes to produce similar pentafluoroethyl copper reagents from pentafluoroethane are multi-step, and suffer from a low atom economy.

### Brief Description of Drawings

Figure 1 shows an X-ray structure of a pentafluoroethylating compound of the present invention, as obtained in Example 3.

### EXAMPLES

The following abbreviations have been used in the Examples:
NMR: Nuclear Magnetic Resonance
GC-MS: Gas chromatography coupled with mass spectrometry
DMF: N,N-dimethylformamide
THF: Tetrahydrofuran
TREAT HF: Triethylamine trihydrofluoride
Py·nHF: Hydrogen fluoride pyridine

Chemicals were purchased from Aldrich (CuCl, TREAT HF, Py·nHF (70% HF)), Alfa Aesar (*tert*-BuOK), and Apollo Scientific (C₂F₅H). All manipulations were performed under argon, unless noted otherwise. THF was distilled from Na/OCPh₂. All solvents including anhydrous DMF (Alfa Aesar or from an MBraun SPS), internal standards for quantitative ¹⁹F NMR analysis, and liquid organic halide reagents were stored over activated 4 A molecular sieves in a glove-box. ¹H, ¹³C , and ¹⁹F NMR spectra were recorded on Bruker Avance 400 Ultrashield and Bruker Avance 500 Ultrashield NMR spectrometers. Quantitative ¹⁹F NMR analyses were carried out with D1 = 5 s. Single-crystal diffraction studies were carried out using a Bruker-Nonius diffractometer equipped with an APEX III 4K CCD area detector. An Agilent Technologies 7890A chromatograph equipped with a 5975C MSD unit was used for GC-MS analysis. All known pentafluoroethylated compounds described in the Examples below exhibited ¹⁹F NMR parameters consistent with those reported in: J. Chem. Soc., Perkin Trans. 1 1980, 661; J. Med. Chem. 2003, 46, 2152; Synth. Commun. 1988, 18, 965; J. Chem. Soc., Perkin Trans. 1 1988, 921; and J. Fluor. Chem. 2010, 131, 212.

### EXAMPLE 1

Under argon, a two-neck 100-mL round-bottom flask equipped with a Teflon-coated magnetic stir-bar was charged with a solution of t-BuOK (purity 97%; 4.72 g; 40.8 mmol), DMF (20 mL), and CuCl (purity 99%; 2.00 g; 20.0 mmol). The reaction mixture was vigorously stirred for 30 min at room temperature. After fluorobenzene (internal standard; 0.56 mL; 6 mmol) was added, C₂F₅H (500 mL; ca. 1.1 equiv) was introduced at vigorous stirring over the period of 1 hour. The reaction mixture was then stirred for an additional hour. To a 0.5-mL aliquot of the thus produced reaction mixture was added 1,3-bis(trifluoromethyl)benzene as an internal standard (10 µL). Quantitative ¹⁹F NMR analysis of the sample indicated that CuC₂F₅ was produced in 95% yield at 0.69 M concentration. The ¹⁹F NMR spectrum showed the formation of CuC₂F₅ (-82.8 ppm, 3F;-110.0 ppm, 2F) and traces amounts (<1%) of [Cu(C₂F₅)₂]⁻ (-82.9 ppm, 3F; -116.1 ppm, 2F).The NMR data are consistent with those reported in: *J. Am. Chem. Soc.* **1986**, *108*, 832 and *Z. Anorg. Allg. Chem.* **2000**, *626*, 999. After slowly stirring the reaction mixture overnight, no change in the yield or concentration of the CuC₂F₅ was observed (¹⁹F NMR). The precipitated KCI was removed by filtration under argon and the solid-free filtrate was transferred to a glass vessel for storage and use in an inert atmosphere.

### EXAMPLE 2

Under argon, CuCl (purity 99%; 6.00 g; 60.0 mmol) was added to a solution of *t*-BuOK (purity 97%; 14.16 g; 122.4 mmol) in DMF (100 mL), and the reaction mixture was vigorously stirred for 30 minutes at room temperature. The precipitated KCI was filtered off and washed on the filter with DMF (20 mL). The combined filtrate and the washings were placed in a 12-oz Fischer-Porter vessel equipped with a pressure gauge, a needle valve, and a Teflon-coated magnetic stir-bar. The vessel was sealed under argon and evacuated to ca. 1 mbar. At vigorous stirring, C₂F₅H was introduced to ca. 10 psi over the period of approximately 5 min. Excess of the gas was then released and the resultant solution (128 mL) was transferred under argon to a glass vessel for storage and use. Quantitative ¹⁹F NMR analysis of a 1-mL aliquot of this solution in the presence of 1,3-bis(trifluoromethyl)benzene as an internal standard (20 µL) indicated that CuC₂F₅ was produced in 91% yield at 0.425 M concentration. The ¹⁹F NMR data were similar to those presented in Example 1.

### EXAMPLE 3

Under argon, a two-neck 100-mL round-bottom flask equipped with a Teflon-coated magnetic stir-bar was charged with t-BuOK (purity 97%; 2.36 g; 20.4 mmol) and a 1:4 v/v mixture of DMF and THF (20 mL). Then CuCl (purity 99%; 1.00 g; 10 mmol) was added and the reaction mixture was vigorously stirred for 30 min at room temperature. After fluorobenzene (internal standard; 0.28 mL; 3 mmol) was added, C₂F₅H (250 mL; ca. 1.1 equiv) was introduced at vigorous stirring over the period of 30 min. The reaction mixture was then stirred for additional 2 hours. Quantitative ¹⁹F NMR analysis of an aliquot of the reaction solution indicated that CuC₂F₅ was produced in 96% yield.The reaction mixture was evaporated under reduced pressure to ca. 4 mL and treated with ether (5 mL). The precipitated KCI was filtered off and washed on filter with ether (2 × 5 mL). The combined filtrate and the washings were treated with hexane (3 mL) and kept at -40 °C for 1 day. The white crystals that precipitated were separated by filtration, washed with a cold 1:1 v/v mixture of ether and hexane, and dried under vacuum. The yield was 1.90 g. The brownish oil at the bottom of the mother liquor was separated, treated with ether (5 mL), and kept at -40°C overnight to give an additional amount (0.83 g) of the product as white crystals that were separated and dried under vacuum. The total yield was 2.73 g (62%). The structure of the product [K(DMF)₂Cu(C₂F₅)(*t*-BuO)] was established by single-crystal X-ray diffraction (Figure 1). ¹H NMR (C₆D₆, 500 MHz): δ = 7.85 (s, 2H), 2.53 (s, 6H), 2.20 (s, 6H), 1.49 (s, 9H). ¹³C NMR (C₆D₆, 126 MHz): δ = 163.2, 141.0 (tq, ¹*J*_{C-F} = 282.9 Hz, ²*J*_{C-F} = 50.4 Hz), 124.3 (qt, ¹*J*_{C-F} = 281.3 Hz, ²*J*_{C-F} = 30.1 Hz), 70.2, 36.8, 35.8, 31.1.

### EXAMPLE 4

To a 15-mL aliquot of the CuC₂F₅ solution prepared as described in Example 1, was added at stirring TREAT HF (Et₃N·3HF; 0.59 mL, 3.63 mmol). After 1 hour, the solid-free solution over the precipitated and settled KF was separated by a syringe. Quantitative ¹⁹F NMR analysis of a 1-mL aliquot of this solution in the presence of 1,3-bis(trifluoromethyl)benzene as an internal standard (20 µL) indicated no change in the concentration of CuC₂F₅ (0.69 M).

### EXAMPLE 5

To a 10-mL aliquot of the solid-free CuC₂F₅ supernatant solution obtained as in Example 4, was added at stirring TREAT HF (Et₃N·3HF; 0.24 mL; 1.48 mmol; 0.2 equiv). Some additional precipitate was produced. After 1 h, the solid-free solution over the settled solid was separated by a syringe and used immediately or stored at -40°C for further use.

### EXAMPLE 6

To an aryl iodide substrate (0.2 mmol) were added a CuC₂F₅ solution prepared as described in Example 5 (0.35 mL; 1.2 equiv) and 1,3-bis(trifluoromethyl)benzene (internal standard; 15.5 µL). This mixture was kept at 23°C or 50°C or first at 23°C and then at 50°C for a certain period of time. The conversions of the CuC₂F₅ and yields of the corresponding pentafluoroethylated products were determined by quantitative ¹⁹F NMR analysis. Conversion of the starting aryl iodide was determined by GC-MS. See Table 1 for specifics.

**Table 1. Pentafluoroethylation of aryl and heteroaryl iodides with CuC₂F₅.**

| | Product | T, °C | time, h | Conversion, % | | Yield, %^{b} |
|---|---|---|---|---|---|---|
| | | | | Arl^{a} | CuC₂F₅^{b} | |
| 1 | 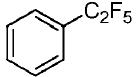 | 50 | 24 | >99 | 88 | 95 |
| 2 | 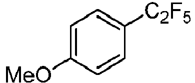 | 23 | 16 | >99 | 88 | 97 |
| | | 50 | 24 | | | |
| 3 | 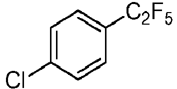 | 23 | 16 | >99 | 88 | 94 |
| | | 50 | 18 | | | |
| 4 | 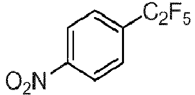 | 23 | 24 | >99 | 86 | 96 |
| 5 | 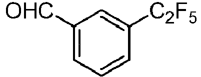 | 23 | 16 | >99 | 88 | 99 |
| | | 50 | 18 | | | |
| 6 | 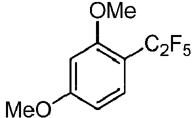 | 23 | 16 | >99 | 88 | 98 |
| | | 50 | 18 | | | |
| 7 | 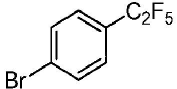 | 23 | 16 | >99 | 92 | 90 |
| | | 50 | 18 | | | |
| 8 | 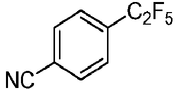 | 23 | 24 | 98 | 85 | 94 |
| 9 | 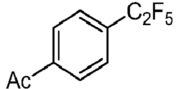 | 23 | 16 | >99 | 87 | 99 |
| | | 50 | 8 | | | |
| 10 | 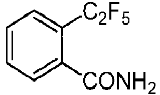 | 23 | 0.5 | >99 | 86 | 92 |
| 11 | 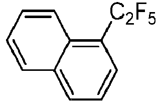 | 23 | 16 | >99 | 87 | 97 |
| | | 50 | 8 | | | |
| 12 | 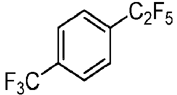 | 23 | 16 | >99 | 88 | 95 |
| | | 50 | 18 | | | |
| 13 | 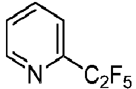 | 23 | 24 | >99 | 86 | 95 |
| 14 | 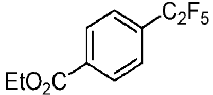 | 23 | 16 | >99 | 86 | 97 |
| | | 50 | 8 | | | |
| 15 | 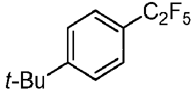 | 23 | 16 | >99 | 86 | 96 |
| | | 50 | 24 | | | |

| | | | | | | |
|---|---|---|---|---|---|---|
| ^{a}Determined by GC-MS. ^{b}Determined by ¹⁹F NMR. | | | | | | |

### EXAMPLE 7

To an aryl bromide substrate (0.2 mmol) were added a CuC₂F₅ solution prepared as described in Example 5 and 1,3-bis(trifluoromethyl)benzene (internal standard; 15.5 µL). This mixture was kept at 80-90°C for a certain period of time. The conversions of the CuC₂F₅ and yields of the corresponding pentafluoroethylated products were determined by quantitative ¹⁹F NMR analysis. Conversion of the starting aryl bromide was determined by GC-MS. See Table 2 for specifics.

**Table 2. Pentafluoroethylation of aryl and heteroaryl bromides with CuC₂F₅.**

| Entry | Product | CuC₂F₅, equiv | T, °C (time, h) | Conversion, % | | Yield, %^{b} |
|---|---|---|---|---|---|---|
| | | | | ArBr^{a} | CuC₂F₅^{b} | |
| 1 | 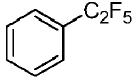 | 2 | 80 (72) | 98 | 93 | 95 |
| 2 | 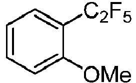 | 1.5 | 90 (24) | >99 | 95 | 99 |
| 3 | 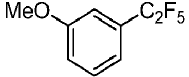 | 2 | 90 (36) | 96 | 98 | 95 |
| 4 | 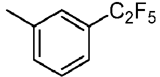 | 2 | 80 (72) | 98 | 95 | 99 |
| 5 | 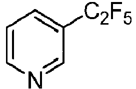 | 2 | 80 (48) | 96 | 95 | 80 |
| 6 | 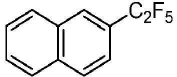 | 2 | 80 (60) | >99 | 90 | 99 (82)^{c} |
| 7 | 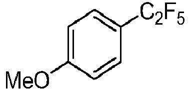 | 2 | 80 (60) | 93 | 95 | 95 |
| 8 | 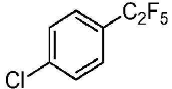 | 2 | 80 (72) | 99 | 92 | 95 |
| 9 | 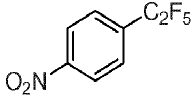 | 1.5 | 80 (18) | >99 | 83 | 99 |
| 10 | 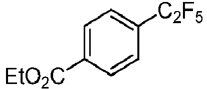 | 1.5 | 80 (36) | >99 | 93 | 99 |

| | | | | | | |
|---|---|---|---|---|---|---|
| ^{a}Determined by GC-MS. ^{b}Determined by ¹⁹F NMR. ^{c}Isolated yield (3 mmol scale). | | | | | | |

### EXAMPLE 8

To 2-bromonaphthalene (purity 97%; 0.64 g; 3 mmol) was added under argon at room temperature a CuC₂F₅ solution prepared as described in Example 5 (conc. = 0.7 M; 8.5 mL; 2 equiv) and the mixture was agitated at 80 °C for 60 h. Ether (30 mL) and water (100 mL) were added at agitation in air. The organic layer was separated and the aqueous layer was washed with ether (2 × 15 mL). The combined ether solutions were washed with brine (2 × 50 mL), dried over MgSO₄, filtered, and evaporated (25 °C, 50 mbar). The residue was dissolved in pentane and the solution was filtered through a short silica gel plug. Evaporation of the filtrate (25°C; 20 mbar) gave crude 2-pentafluoroethylnaphthalene as a white solid (0.72 g, 97%). Recrystallization from methanol and subsequent drying under vacuum (25°C, 1 mbar; partial sublimation of the product was observed) gave pure 2-pentafluoroethylnaphthalene (0.62 g; 82%). ¹H NMR (CDCl₃, 400 MHz): δ = 8.14 (s, 1 H), 8.00 - 7.88 (m, 3H), 7.67 - 7.55 (m, 3H). ¹³C NMR (CDCl₃, 100 MHz): δ = 134.8 (t, ⁴*J*_{C-F} = 1.4 Hz), 132.4, 129.0, 128.4, 128.0, 127.5 (t, ³*J*_{C-F} = 7.1 Hz), 127.3, 126.1 (t, ²*J*_{C-F} = 23.9 Hz), 122.4 (tq, ³*J*_{C-F} = 5.7 Hz, ⁴*J*_{C-F} = 0.9 Hz), 119.4 (qt, ¹*J*_{C-F} = 286.0 Hz, ²*J*_{C-F} = 39.5 Hz), 113.8 (tq, ¹*J*_{C-F} = 253.7 Hz, ²*J*_{C-F} = 38.2 Hz). ¹⁹F NMR (CDCl₃, 376 MHz): δ = -84.60 (s, 3F), -114.43 (s, 2F). Anal. Calcd. for C₁₂H₇F₅: C, 58.6; H, 2.9. Found: C, 59.1; H, 3.1.

### EXAMPLE 9

To a solution of K[(*t*-BuO)₂CU] prepared as described in *J*. *Am. Chem. Soc.* **2011**, *133,* 20901 (0.5 mL; 0.25 mmol) were added 1,3-bis(fluoromethyl)bezene (internal standard; 19 µL). Pentafluoroethane (20 mL) was bubbled through this solution at room temperature. Then 4-fluoroiodobenzene (0.29 mL; 10 equiv) was added and the mixture was heated at 80°C. After 15 hours, quantitative conversion of the CuC₂F₅ reagent to a 1:1.2 mixture of 4-C₂F₅C₆H₄F and 4-*t*-BuOC₆H₄F was observed by ¹⁹F NMR.

### EXAMPLE 10

The experiment described in Example 9 was repeated, except TREAT HF (1 mol HF per 1 mol of Cu) was added 5-10 minutes after the addition of C₂F₅H and before the addition of 4-fluoroiodobenzene. After heating the thus prepared reaction mixture at 80°C for 40 minutes, full conversion of the CuC₂F₅ reagent was observed by ¹⁹F NMR. The yield of 4-C₂F₅C₆H₄F was 88%. No formation of 4-*t*-BuOC₆H₄F was observed in this case.

### EXAMPLE 11

To a CuC₂F₅ solution prepared as in Example 2 (0.5 mL) were added 1,3-bis(trifluoromethyl)benzene (internal standard; 19 µL; 0.125 mmol) and acetic acid (14 µL), which resulted in quick precipitation of white crystals (KOAc). After 10 minutes, the resultant solution was analyzed by ¹⁹F NMR. The ¹⁹F NMR chemical shifts of CuC₂F₅ after the reaction with AcOH were -83.1 and -111.1 ppm and for [Cu(CF₂CF₃)₂]⁻ (ca. 4%)-82.9 and -115.9 ppm. After 4-fluoroiodobenzene (0.29 mL; 10 equiv) was added and the mixture was heated at 50°C for 15 hours, 4-C₂F₅C₆H₄F was produced in 85% yield (¹⁹F NMR). No t-butoxylation was observed.

### EXAMPLE 12

The experiment described in Example 11 was repeated, except acetic acid was replaced with HCl in dioxane (4M; 0.125 mL). After heating the reaction mixture at 50°C for 15 hours, full conversion of the CuC₂F₅ reagent was observed by ¹⁹F NMR. The yield of 4-C₂F₅C₆H₄F was 90%. No formation of 4-t-BuOC₆H₄F was observed in this case.

### EXAMPLE 13

Pentafluoroethane (20 mL) was bubbled through a solution of [K(DMF)][Cu(OBu-t)₂] (80 mg; 0.25 mmol; prepared as described in J. Am. Chem. Soc. 2011, 133, 20901) and 1,3-bis(trifluoromethyl)benzene (internal standard; 19 µL; 0.125 mmol) in THF (0.5 mL) at room temperature (ca. 23°C). ¹⁹F NMR analysis after 10 min indicated the formation of CuC₂F₅ as the only product in 81% yield. No formation of [Cu(CF₂CF₃)₂]⁻ was observed. No decomposition of the CuC₂F₅ in this solution was observed after 18 hours at 23°C (¹⁹F NMR). 4-Fluoroiodobenzene (10 equiv, 0.29 mL) was added and the reaction mixture was heated for 1 hour at 50°C. Approximately one third of the CuC₂F₅ had decomposed and only trace amounts of 4-C₂F₅C₆H₄F and 4-*t*-BuOC₆H₄F were produced (¹⁹NMR). Then, acetic acid (14 µL) was added (white precipitate of KOAc was produced) and the mixture was heated at 50 °C for 15 h. Full conversion of the remaining CuC₂F₅ to 4-C₂F₅C₆H₄F (50% yield) was observed (¹⁹F NMR). These data indicate that after the treatment with AcOH, nearly all of the CuC₂F₅ that had remained was converted to 4-C₂F₅C₆H₄F.

### EXAMPLE 14

An 30-mL aliquot of the CuC₂F₅ solution prepared as described in Example 2 was treated with acetic acid (0.8 mL; 14.06 mmol; 1 equiv). The precipitated KOAc was filtered off. ¹⁹F NMR analysis of a 1-mL aliquot of the filtrate in the presence of 1,3-bis(trifluoromethyl)benzene as an internal standard (20 µL) indicated no change in the concentration of the CuC₂F₅ (0.425 M). The solution was stable for at least 5 days at room temperature (¹⁹F NMR).

### EXAMPLE 15

To a 20-mL aliquot of the CuC₂F₅ solution prepared as described in Example 2, was added at stirring Py·nHF (ca. 70% HF; 244 µL; 9.38 mmol) to produce a white precipitate of KF. After 1 hour, the solid-free solution over the settled KF was separated by a syringe. Quantitative ¹⁹F NMR analysis of a 1-mL aliquot of this solution in the presence of 1,3-bis(trifluoromethyl)benzene as an internal standard (20 µL) indicated no change in the concentration of CuC₂F₅ (0.425 M).

### EXAMPLE 16

To a 5-mL aliquot of the solid-free CuC₂F₅ supernatant solution obtained as in Example 15, was added at stirring Py·nHF (61 µL; 2.35 mmol; 1 equiv). Some additional precipitate was produced. After 1 hour, the solid-free solution over the settled solid was separated by a syringe and used immediately or stored at -40°C for further use.

### EXAMPLE 17

To a 30-mL aliquot of the CuC₂F₅ solution prepared as described in Example 2, was added at stirring TREAT HF (Et₃N·3HF; 0.76 mL, 4.67 mmol). After 1 hour, the solid-free solution over the precipitated and settled KF was separated by a syringe. Quantitative ¹⁹F NMR analysis of a 1-mL aliquot of this solution in the presence of 1,3-bis(trifluoromethyl)benzene as an internal standard (20 µL) indicated no change in the concentration of CuC₂F₅ (0.425 M).

### EXAMPLE 18

To a 10-mL aliquot of the solid-free CuC₂F₅ supernatant solution obtained as in Example 17, was added at stirring TREAT HF (Et₃N·3HF; 152 µL; 0.93 mmol; 0.2 equiv). Some additional precipitate was produced. After 1 hour, the solid-free solution over the settled solid was separated by a syringe and used immediately or stored at -40°C for further use.

### EXAMPLE 19

To a 5-mL aliquot of the solid-free CuC₂F₅ supernatant solution obtained as in Example 17, was added at stirring TREAT HF (Et₃N·3HF; 252 µL; 1.55 mmol; 0.66 equiv). Some additional precipitate was produced. After 1 h, the solid-free solution over the settled solid was separated by a syringe and used immediately or stored at -40°C for further use.

### EXAMPLE 20

To 2-bromoacetophenone (purity 98%; 25.4 mg; 0.125 mmol) were added a solution of CuC₂F₅ obtained as in Example 17 (0.425 M; 0.44 mL; 1.5 equiv) and 1,3-bis(trifluoromethyl)benzene (internal standard; 9.7 µL). After 20 min at room temperature, GC-MS and quantitative ¹⁹F NMR analysis indicated the formation of 1,4,4,4-tetrafluoro-1-phenylbut-2-en-1-one in 80% yield at >99% conversion of 2-bromoacetophenone and no formation of 3,3,4,4,4-pentafluoro-1-phenylbutan-1-one.

### EXAMPLE 21

To 2-bromoacetophenone (purity 98%; 25.4 mg; 0.125 mmol) were added a solution of CuC₂F₅ obtained as in Example 19 (0.425 M; 0.44 mL; 1.5 equiv) and 1,3-bis(trifluoromethyl)benzene (internal standard; 9.7 µL). After 40 min at room temperature, GC-MS and quantitative ¹⁹F NMR analysis indicated the formation of 3,3,4,4,4-pentafluoro-1-phenylbutan-1-one (25%) and 3,4,4,4-tetrafluoro-1-phenylbut-2-en-1-one (30%) at >99% conversion of 2-bromoacetophenone.

### EXAMPLE 22

To benzyl bromide (purity >98%; 15 µL; 0.125 mmol) were added a CuC₂F₅ solution obtained as in Example 16 (0.425 M; 0.44 mL; 1.5 equiv) and 1,3-bis(trifluoromethyl)benzene (internal standard; 9.7 µL). After 24 hours at room temperature, GC-MS and quantitative ¹⁹F NMR analysis indicated the formation of 2,2,3,3,3-pentafluoropropylbenzene in 78% yield at >99% conversion of benzyl bromide.

### EXAMPLE 23

To β-bromostyrene (cis/trans = 1:8; purity 96%; 17 µL; 0.125 mmol) were added a CuC₂F₅ solution obtained as in Example 18 (0.425 M; 0.44 mL; 1.5 equiv) and 1,3-bis(trifluoromethyl)benzene (internal standard; 9.7 µL). After 63 hours at room temperature, GC-MS and quantitative ¹⁹F NMR analysis indicated the formation of (3,3,4,4,4-pentafluorobut-1-en-1-yl)benzene as a mixture of *E* (85%) and *Z* (11%) isomers at >99% conversion of β-bromostyrene.

### EXAMPLE 24

To cis/trans-2-bromobut-2-ene (purity 98%; 13 µL; 0.125 mmol) were added a CuC₂F₅ solution obtained as in Example 18 (0.425 M; 0.44 mL; 1.5 equiv) and 1,3-bis(trifluoromethyl)benzene (internal standard; 9.7 µL). After 32 h at 50°C, GC-MS and quantitative ¹⁹F NMR analysis indicated the formation of 4,4,5,5,5-pentafluoro-3-methylpent-2-ene as a mixture of *E* (42%) and *Z* (39%) isomers at 95% conversion of 2-bromobut-2-ene.

### EXAMPLE 25

To p-toluoyl chloride (purity 98%; 17 µL; 0.125 mmol) were added a CuC₂F₅ solution obtained as in Example 15 but containing extra 0.6 equiv of HF in the form of Py·nHF (0.425 M; 0.44 mL; 1.5 equiv) and 1,3-bis(trifluoromethyl)benzene (internal standard; 9.7 µL). After 35 min at room temperature, GC-MS and quantitative ¹⁹F NMR analysis indicated the formation of 2,2,3,3,3-pentafluoro-1-(p-tolyl)propan-1-one in 30% yield.

### EXAMPLE 26

In air, to 4-biphenylylboronic acid (24.8 mg; 0.125 mmol) was added a CuC₂F₅ solution obtained as in Example 17 (0.425 M; 0.59 mL; 2 equiv). After stirring the mixture at room temperature in air for 1 hour, 1,3-bis(trifluoromethyl)benzene (internal standard; 9.7 µL) was added. GC-MS and quantitative ¹⁹F NMR analysis indicated the formation of 4-(pentafluoroethyl)-1,1'-biphenyl was produced in 78% yield.

### EXAMPLE 27

In air, to ethynylbenzene (purity >97%; 14 µL; 0.125 mmol) were added a CuC₂F₅ solution obtained as in Example 17 (0.425 M; 0.59 mL; 2 equiv) and 1,3-bis(trifluoromethyl)benzene (internal standard; 9.7 µL). After stirring the mixture at room temperature in air for 1 hour, GC-MS and quantitative ¹⁹F NMR analysis indicated the formation of pentafluoroethyl(phenyl)acetylene in 68% yield.

### EXAMPLE 28

To a solution of triphenylphosphine (purity 99%; 1.84 g; 7 mmol; 5 equiv) in ether (10 mL) was added drop-wise, at agitation, a solution of CuC₂F₅ in DMF prepared as described in Example 4 (2 mL; 1 equiv). After stirring the reaction mixture for 30 min, hexane (10 mL) and ether (20 mL) were added and the solid-free solution was kept at -40°C for 1 day. The resulting white crystals were quickly separated from the cold solution, washed with ether (10 mL), and dried under vacuum. The structure [(Ph₃P)₂CuC₂F₅] has been established by X-ray analysis.The yield was 0.65 g (66%). ¹⁹F NMR (CH₂Cl₂, 376 MHz): δ = -82.5 (br s, 3F), -110.5 (br s, 2F). A minor quantity of [Cu(C₂F₅)₂]⁻ was also detected.

### EXAMPLE 29

To [(tmeda)Pd(Ph)I] (0.3 g; 0.7 mmol) was added a solution of CuC₂F₅ in DMF prepared as described in Example 4 (1 mL; 1.1 equiv). After stirring the reaction mixture for 48 h at 50°C, the product was isolated in air. The mixture was diluted with ether (30 mL), stirred for 1 hour in air, filtered through Celite, evaporated to ca. 1 mL, treated with methanol (20 mL), and kept at -20°C overnight. The precipitated white crystals of [(tmeda)Pd(Ph)(C₂F₅)] were separated, washed with methanol, and dried under vacuum. The yield was 0.11 g. Evaporation of the combined mother liquor and the washings produced additional 0.15 g of the pure product on washing with methanol and drying. Total yield: 0.26 g (90%). ¹H NMR (CD₂Cl₂, 400 MHz): δ = 7.40 (d, *J* = 7.3 Hz, 2H), 6.96 - 6.88 (m, 2H), 6.89 - 6.82 (m, 1 H), 2.62 (s, 6H), 2.62 - 2.49 (m, 4H), 2.12 (s, 6H). ¹³C NMR (CD₂Cl₂, 100 MHz): δ = 156.8 (tq, ³*J*_{C-F} = 5.9 Hz, ⁴*J*_{C-F} = 1.4 Hz), 137.1, 129.5 (tq, ¹*J*_{C-F} = 299.9 Hz, ²*J*_{C-F} = 44.9 Hz), 126.3, 123.0, 122.4 (qt, ¹*J*_{C-F} = 285.0 Hz, ²*J*_{C-F} = 32.5 Hz), 61.0, 60.5, 49.4, 49.1. ¹⁹F NMR (CD₂Cl₂, 376 MHz): δ = -80.6 (s, 3F), -96.9 (s, 2F). Anal. Calcd. for C₁₄H₂₁F₅N₂Pd: C, 40.2; H, 5.1; N, 6.7. Found: C, 39.9; H, 4.9; N, 6.7.

The following numbered clauses are preferred statements of the invention, and part of the description.
1. A method of obtaining a pentafluoroethylating composition comprising reacting a cuprating agent comprising the [Cu(OR¹)(OR²)] moiety with pentafluoroethane, wherein:
   Cu is in the oxidation state of +1; and
   R¹ is C₁₋₂₀ alkyl or C₅₋₂₀ aryl, wherein C₁₋₂₀ alkyl is optionally substituted with one or more groups selected from: C₅₋₂₀ aryl, C₁₋₂₀ alkoxy and C₅₋₂₀ aryloxy, and wherein C₅₋₂₀ aryl is optionally substituted with one or more groups selected from: C₁₋₂₀ alkyl, C₁₋₂₀ alkoxy, C₅₋₂₀ aryl, C₅₋₂₀ aryloxy, halo, nitro and cyano; and
   R² is C₁₋₂₀ alkyl or C₅₋₂₀ aryl, wherein C₁₋₂₀ alkyl is optionally substituted with one or more groups selected from: C₅₋₂₀ aryl, C₁₋₂₀ alkoxy and C₅₋₂₀ aryloxy, and wherein C₅₋₂₀ aryl is optionally substituted with one or more groups selected from: C₁₋₂₀ alkyl, C₁₋₂₀ alkoxy, C₅₋₂₀ aryl, C₅₋₂₀ aryloxy, halo, nitro and cyano.
2. The method according to clause 1, wherein R¹ and R² are independently unsubstituted C₁₋₂₀ alkyl.
3. The method according to clause 2, wherein R¹ and R² are independently unsubstituted C₁₋₁₀ alkyl.
4. The method according to clause 3, wherein R¹ and R² are independently unsubstituted C₁₋₇ alkyl.
5. The method according to clause 4, wherein R¹ and R² are independently selected from the group consisting of methyl, ethyl, propyl, iso-propyl, butyl, iso-butyl, tert butyl, pentyl and neo-pentyl.
6. The method according to clause 4, wherein R¹ and R² are independently methyl or *tert*-butyl.
7. The method according to clause 4, wherein R¹ and R² are *tert*-butyl.
8. The method according to any one of clauses 1 to 6 wherein R¹ and R² are the same.
9. The method according to any one of clauses 1 to 6 wherein R¹ and R² are different.
10. The method according to any of the preceding clauses that is carried out in an anhydrous aprotic solvent.
11. The method according to clause 10, wherein the anhydrous aprotic solvent is selected from the group consisting of N,N-dimethylformamide (DMF), tetrahydrofurane (THF), 1,4-dioxane, 1,3-dimethyl-2-imidazolidinone (DMI), N,N-dimethylacetamide (DMAC), 1,3-dimethyl-3,4,5,6-tetrahydro-2(1H)-pyrimidinone (DMPU), N-methylpyrrolidone (NMP), dimethylsulfoxide (DMSO), tetramethylurea (TMU), hexamethylphosphoramide (HMPA) and mixtures thereof.
12. The method according to clause 11, wherein the anhydrous aprotic solvent is selected from anhydrous N,N-dimethylformamide (DMF), anhydrous N-methylpyrrolidone (NMP) and mixtures thereof.
13. The method according to clause 12, wherein the anhydrous aprotic solvent is N,N-dimethylformamide (DMF).
14. The method according to any of the preceding clauses which is performed by contacting the reactants at stirring for from 1 second to 48 hours.
15. The method according to clause 14, which is performed by contacting the reactants at stirring for from 1 minute to 4 hours.
16. The method according to any of the preceding clauses which is carried out at a temperature from -30°C to 100°C.
17. The method according to clause 16, which is carried out at a temperature from -30°C to 80°C.
18. The method according to clause 17, which is carried out at a temperature from -10°C to 50°C
19. The method according to clause 18, which is carried out at a temperature from 15°C to 30°C
20. The method according to clause 19, which is carried out at room temperature.
21. The method according to any of the preceding clauses which is carried out under a pressure from 0.1 to 50 bar.
22. The method according to clause 21, which is carried out under atmospheric pressure.
23. The method according to any one of the preceding clauses wherein the concentration of the cuprating agent is comprised from 0.05 to 4 M.
24. The method according to clause 23 wherein the concentration of the cuprating agent is comprised from 0.3 to 0.7 M.
25. The method according to any one of the preceding clauses wherein the cuprating agent comprising the [Cu(OR¹)(OR²)] moiety is a compound of formula (**II**) or a polymer or oligomer thereof:

   MₗSₘCuₓ(OR¹)_{y}(OR₂)_{z}X_{q} (**II**)

   wherein:
   M is selected from the group consisting of lithium, sodium, potassium, rubidium, cesium, calcium, barium and magnesium; and
   S is selected from N,N-dimethylformamide (DMF), 1,3-dimethyl-2-imidazolidinone (DMI), N,N-dimethylacetamide (DMAC), 1,3-dimethyl-3,4,5,6-tetrahydro-2(1H)-pyrimidinone (DMPU), N-methylpyrrolidone (NMP), dimethylsulfoxide (DMSO), tetramethylurea (TMU),
   and hexamethylphosphoramide (HMPA);
   R¹ and R² are as defined in any of clauses 1 to 9;
   X is chloride, bromide or iodide;
   I is an integer from 1 to 20;
   m is an integer from 0 to 20;
   x, y, and z are each an integer from 1 to 20;
   q is an integer from 0 to 10; and where:
   the sum of y and z is equal to twice the value of x; and
   the sum of I and x is equal to the sum of y, z, and q when M is selected from lithium, sodium, potassium, rubidium and cesium; or
   the sum of twice the value of I and x is equal to the sum of y, z, and q when M is selected from calcium, magnesium and barium.
26. The method according to clause 25, wherein M is selected from sodium, potassium, rubidium and cesium.
27. The method according to clause 26, wherein M is sodium or potassium.
28. The method according to clause 27, wherein M is potassium.
29. The method according to any one of clauses 25 to 28, wherein S is selected from N,N-dimethylformamide (DMF), 1,3-dimethyl-2-imidazolidinone (DMI), N-methylpyrrolidone (NMP), hexamethylphosphoramide (HMPA), and N,N-dimethylacetamide (DMAC).
30. The method according to clause 29, wherein S is N,N-dimethylformamide (DMF).
31. The method according to any one of clauses 25 to 30, wherein q is an integer between 0 and 2.
32. The method according to any one of clauses 25 to 31, wherein X is chloride.
33. The method according to any one of clauses 25 to 31, wherein X is bromide.
34. The method according to either clause 32 or clause 33, wherein q is 0.
35. The method according to any one of clauses 25 to 31, wherein X is iodide.
36. The method according to clause 35, wherein q is 2.
37. The method according to any one of clauses 25 to 36, wherein m is an integer between 0 and 10.
38. The method according to clause 37, wherein m is an integer between 0 and 8.
39. The method according to any one of clauses 27 to 38, wherein m is an integer between 1 and 20.
40. The method according to clause 39, wherein m is an integer between 1 and 10.
41. The method according to clause 40, wherein m is an integer between 1 and 8.
42. The method according to any one of clauses 25 to 41, wherein I is an integer between 1 and 8.
43. The method according to any one of clauses 25 to 41, wherein x, y, and z are an integer between 1 and 8.
44. The method according to clause 43, wherein x, y and z are the same and are an integer between 1 and 6.
45. The method according to clause 25, wherein the cuprating agent is selected from IK₈Cu₆(*tert*-BuO)₁₂(DMF)₈I]I; K(DMF)[Cu(*tert*-BUO)₂]; Na(DMF)₂[Cu(*tert*-BuO)₂] and polymers or oligomers thereof.
46. The method according to any one of the preceding clauses, further comprising the initial step of preparing the cuprating agent by contacting a copper (I) source with a base in the presence of a solvent, wherein:
   the base is selected from the compounds of formula (**I**) and mixtures thereof

   M-OR¹ (**I**);

   wherein M is selected from the group consisting of lithium, sodium, potassium, rubidium, cesium, calcium, barium and magnesium.
47. The method according to clause 46, wherein the base is selected from potassium tert-butaxide, potassium methoxide and mixtures thereof.
48. The method according to clause 47, wherein the base is potassium tert-butoxide.
49. The method according to any one of clauses 46 to 48, wherein the the copper (I) source is generated in situ from a copper (II) or a copper (III) compound and a reducing agent.
50. The method according to any one of clauses 46 to 48, wherein the copper (I) source is generated in situ from metallic copper and an oxidizing agent.
51. The method according to any one of clauses 46 to 48, wherein the copper (I) source is selected from copper (I) chloride, copper (I) bromide, copper (I) iodide, copper (I) cyanide, copper (I) oxide, copper (I) trifluoromethanesulfonate, copper (I) thiocyanate, [Cu[1,3-bis(2,6-diisopropylphenyl)imidazol-2-ylidene]Cl, [Cu[1,3-bis(2,6-diisopropylphenyl)imidazol-2-ylidene]Br, [CuBr(1,10-phenanthroline)]₂, [CuCl(1,10-phenanthroline)]₂, [Cul(1,10-phenanthroline)]2, copper (I) trifluoroacetate, Cu(OR³), Cu(OCOR³), Cu(SR³), Cu(SR³₂)Br, Cu(SR³₂)Cl, Cu(SR³₂)I, Cu(OSO₂R³) and mixtures thereof, wherein R³ is C₁₋₂₀ alkyl or C₅₋₂₀ aryl, wherein C₁₋₂₀ alkyl is optionally substituted with one or more groups selected from: C₅₋₂₀ aryl, C₁₋₂₀ alkoxy and C₅₋₂₀ aryloxy, and wherein C₅₋₂₀ aryl is optionally substituted with one or more groups selected from: C₁₋₂₀ alkyl, C₁₋₂₀ alkoxy, C₅-₂₀ aryl, C₅₋₂₀ aryloxy, halo, nitro and cyano.
52. The method according to clause 51, wherein the copper (I) source is selected from copper (I) chloride, copper (I) bromide, copper (I) iodide, copper (I) cyanide, copper (I) oxide, copper (I) trifluoromethanesulfonate, copper (I) thiocyanate, [Cu[1,3-bis(2,6-diisopropylphenyl)imidazol-2-ylidene]Cl, [Cu[1,3-bis(2,6-diisopropylphenyl)imidazol-2-ylidene]Br, [CuBr(1,10-phenanthroline)]₂, [CuCl(1,10-phenanthroline)]₂, [Cul(1,10-phenanthroline)]₂, copper (I) trifluoroacetate, Cu(OCOR³), Cu(SR³), Cu(SR³₂)Br, Cu(SR³₂)Cl, Cu(SR³₂)I, Cu(OSO₂R³) and mixtures thereof.
53. The method according to clause 52, wherein the copper (I) source is selected from copper (I) chloride, copper (I) bromide, copper iodide, copper (I) acetate, copper (I) trifluoromethanesulfonate, and mixtures thereof.
54. The method according to clause 52, wherein the copper (I) source is selected from copper (I) chloride, copper (I) bromide, copper iodide and mixtures thereof.
55. The method according to any one of clauses 46 to 54, wherein the solvent in the initial step of producing the cuprating agent is a polar aprotic solvent.
56. The method according to clause 55, wherein the solvent is selected from the group consisting of: N,N-dimethylformamide (DMF), 1,3-dimethyl-2-imidazolidinone (DMI), N-dimethylacetamide (DMAC), 1,3-dimethyl-3,4,5,6-tetrahydro-2(1 H)-pyrimidinone (DMPU), N-methylpyrrolidone (NMP), dimethylsulfoxide (DMSO), tetramethylurea (TMU), hexamethylphosphoramide (HMPA), and mixtures thereof.
57. The method according to any one of clauses 46 to 54, wherein the solvent in the initial step is the same as S in the compound of formula **II**.
58. The method according to any one of clauses 46 to 57, wherein the initial step is performed by contacting the reactants at stirring for from 1 second to 48 hours.
59. The method according to clause 58, wherein the initial step is performed by contacting the reactants at stirring for from 1 minute to 4 hours.
60. The method according to any one of clauses 46 to 58, wherein the initial step is carried out at a temperature from -30°C to 100°C.
61. The method according to clause 60, wherein the initial step is carried out at a temperature from -30°C to 80°C.
62. The method according to clause 60, wherein the initial step is carried out at a temperature from -10°C to 50°C
63. The method according to clause 62, wherein the initial step is carried out at a temperature from 15°C to 30°C
64. The method according to clause 63, wherein the initial step is carried out at room temperature.
65. The method according to any one of clauses 46 to 57, wherein the initial step is carried out under a pressure from 0.1 to 50 bar.
66. The method according to clause 65, wherein the initial step is carried out under atmospheric pressure.
67. The method according to any one of the clauses 46 to 48 and 52 to 54 wherein the amount of base is 2 gram-moles per each gram-atom of copper (I) in the copper (I) source.
68. The method of any one of the preceding clauses further comprising the step of treating pentafluoroethylating composition obtained by reacting the cuprating agent with pentafluoroethane with an acid.
69. The method according to clause 68, wherein the acid is selected from hydrogen fluoride, hydrogen chloride, acetic acid, trifluoroacetic acid, formic acid, hydrogen fluoride pyridine, mixtures of compounds of formula NR³R⁴R⁵ with hydrogen fluoride, melamine/HF, poly[4-vinylpyridinium poly(hydrogen fluoride)], mixtures thereof and B(OR⁶)₃; wherein R³, R⁴, R⁵ and R⁶ are independently unsubstituted C₁₋₂₀ alkyl.
70. The method according to clause 69, wherein the acid is is selected from the group consisting of hydrogen fluoride, hydrogen fluoride pyridine, hydrogen chloride, acetic acid, triethylamine trihydrofluoride, melamine/HF, poly[4-vinylpyridinium poly(hydrogen fluoride)], mixtures thereof and B(OR⁶)₃₋
71. The method according to clause 70, wherein the acid is selected from the group consisting of hydrogen fluoride, hydrogen fluoride pyridine, hydrogen chloride, acetic acid, triethylamine trihydrofluoride, mixtures thereof and B(OR⁶)₃.
72. The method according to clause 71, wherein the acid is selected from the group consisting of hydrogen fluoride, hydrogen fluoride pyridine, hydrogen chloride, acetic acid, triethylamine trihydrofluoride, mixtures thereof and B(OCH₃)₃.
73. The method according to clause 72, wherein the acid is triethylamine trihydrofluoride.
74. The method according to clause 72, wherein the acid is hydrogen fluoride pyridine.
75. The method according to clause 72, wherein the acid is hydrogen chloride.
76. The method according to clause 72, wherein the acid is is acetic acid.
77. The method according to any one of clauses 68 to 76, wherein the acid is added neat.
78. The method according to any one of clauses 68 to 76, wherein the acid is added in solution.
79. The method according to clause 78, wherein the the solvent in which the acid is added is selected from the group consisting of N,N-dimethylformamide (DMF), 1,3-dimethyl-2-imidazolidinone (DMI), N,N-dimethylacetamide (DMAC), 1,4-dioxane, tetrahydrofuran, 1,3-dimethyl-3,4,5,6-tetrahydro-2(1H)-pyrimidinone (DMPU), N-methylpyrrolidone (NMP), dimethylsulfoxide (DMSO), tetramethylurea (TMU), hexamethylphosphoramide (HMPA) and mixtures thereof.
80. The method according to clause 79, wherein the the solvent in which the acid is added is selected from anhydrous N,N-dimethylformamide (DMF), anhydrous N-methylpyrrolidone (NMP) and mixtures thereof.
81. The method according to clause 78, wherein the the solvent in which the acid is added is the solvent used in the reaction of the cuprating agent with pentafluroethane.
82. The method according to any one of clauses 68 to 81, wherein after the treatment of the pentafluoroethylating composition with an acid, the alcohol produced is removed from the mixture under vacuum, via adsorption or via chemisorption.
83. A pentafluroethylating composition obtainable by the method according to any one of clauses 1 to 82.
84. A pentafluroethylating composition according to clause 83 comprising the [Cu(C₂F₅)(OR¹)] moiety.
85. A pentafluroethylating composition comprising the [Cu(C₂F⁵)(OR¹)] moiety, wherein R¹ is C₁₋₂₀ alkyl or C₅₋₂₀ aryl, wherein C₁₋₂₀ alkyl is optionally substituted with one or more groups selected from: C₅₋₂₀ aryl, C₁₋₂₀ alkoxy and C₅₋₂₀ aryloxy, and wherein C₅₋₂₀ aryl is optionally substituted with one or more groups selected from: C₁₋₂₀ alkyl, C₁₋₂₀ alkoxy, C₅₋₂₀ aryl, C₅₋₂₀ aryloxy, halo, nitro and cyano.
86. The pentafluoroethylating composition of clause 85 that is a compound of formula (**III**) or a poymer of oligomer thereof:

   M_{l'}S_{m'}Cu_{x'}(OR¹)_{y'}(C₂F₅)_{z}'X_{q'} (**III**)

   wherein:
   Cu is in the oxidation state +1;
   M, S, R¹ and X are as defined in any one of clauses 1 to 45;
   I' is an integer from 1 to 20;
   m' is an integer from 0 to 20;
   x', y' and z' are each an integer from 1 to 20;
   q' is an integer from 0 to 10; and where:
   the sum of y' and z' is equal to twice the value of x'; and
   the sum of I' and x' is equal to the sum of y', z' and q' when M is selected from lithium, sodium, potassium, rubidium and cesium; or, alternatively,
   the sum of twice the value of I' and x' is equal to the sum of y', z' and q' when M is selected from calcium, magnesium and barium.
87. The pentafluoroethylating composition of clause 86, wherein q' is an integer between 0 and 2.
88. The pentafluoroethylating composition according to clause 87, wherein q' is 0.
89. The pentafluoroethylating composition according to any one of clauses 86 to 88, wherein m' is an integer between 0 and 10.
90. The pentafluoroethylating composition according to clause 89, wherein m' is an integer between 0 and 5.
91. The pentafluoroethylating composition according to any one of clauses 86 to 90, wherein I' is an integer between 1 and 8.
92. The pentafluoroethylating composition according to clause 91, wherein I' is 1.
93. The pentafluoroethylating composition according to any one of clauses 86 to 92, wherein x', y' and z' are each an integer between 1 and 8.
94. The pentafluoroethylating composition according to clause 93, wherein x', y' and z' are 1.
95. The pentafluoroethylating composition according to clause 86 wherein
   M is selected from sodium, potassium, and cesium;
   q' is 0_{;}
   m' is an integer from 0 to 5;
   l' is 1; and
   x', y' and z' are the same and are 1.
96. The pentafluoroethylating composition according to clause 86 which is K(DMF)₂Cu(O-*tert*-Bu)(C₂F₅), a polymer or an oligomer thereof.
97. Use of a pentafluoroethylating composition as defined in any one of clauses 83 to 96 in the transfer of a pentafluoroethyl group to a substrate selected from an electrophile, a terminal alkyne, or to the carbon atom of an organoboron compound.
98. The use of a pentafluoroethylating composition according to clause 97, wherein the organoboron compound is of formula (**IV**):

   R^{BN}-B(OH)₂ (**IV**)

   where R^{BN} is selected from optionally susbtitued C₅₋₂₀ aryl, C₁₋₂₀ alkyl and C₃₋₂₀ heterocyclyl.
99. The use of a pentafluoroethylating composition according to clause 97, wherein the electrophile is selected from: C₅₋₂₀ aryl halides, C₃₋₂₀ heterocyclyl halides, C₁₋₂₀ alkyl halides, acyl halides, C₅₋₂₀ aryl sulfonates, C₃₋₂₀ heterocyclyl sulfonates, C₁₋₂₀ alkyl sulfonates, electron-deficient derivatives of Si, Ge, B, P, As, Sb, Bi, S, Se, Te and a metal atom in a metal compound.
100. The use of a pentafluoroethylating composition according to clause 99, wherein the electrophile is selected from the group consisting of: C₅₋₂₀ aryl halides, C₃₋₂₀ heterocyclyl halides, C₁₋₂₀ alkyl halides, acyl halides, C₅₋₂₀ aryl sulfonates, C₃₋₂₀ heterocyclyl sulfonates and C₁₋₂₀ alkyl sulfonates.
101. The use of a pentafluoroethylating composition according to clause 97, wherein the elctrophile is a metal atom in a metal compound, and the metal is selected from Pd, Pt, Ni, Zn, Cd, Co, Fe, Ru, Rh and Ir.
102. The use of a pentafluoroethylating composition according to clause 97, wherein the terminal alkyne is of formula (**V**):

   R^{AL}-C=C-H (**V**)

   where R^{AL} is selected from optionally susbtitued C₅₋₂₀ aryl, C₁₋₂₀ alkyl and C₃₋₂₀ heterocyclyl.
103. The use according to any one of clauses 97 to 102, wherein the pentafluoroethylating composition is used at a temperature from -30°C to 180°C.
104. The use according to clause 103, wherein preferably the pentafluoroethylating composition is used at a temperature from -30°C to 90°C.
105. The use according to clause 104, wherein preferably the pentafluoroethylating composition is used at a temperature from 20°C to 90°C.
106. The use according to clause 105, wherein preferably the pentafluoroethylating composition is used at a temperature from 25°C to 90°C.
107. The use according to any one of clauses 97 to 106, wherein the pentafluoroethylating composition is reacted for from 1 minute to 30 days.
108. The use according to any one of clauses 97 to 107, wherein the pentafluoroethylating composition is reacted for from 1 minute to 72 hours.
109. The use according to any one of clauses 97 to 108, wherein the pentafluoroethylating composition is reacted under a pressure from 0.1 to 50 bar.
110. The use according to clause 109, wherein the pentafluoroethylating composition is reacted under atmospheric pressure.
111. The use of the pentafluoroethylating composition as defined in any one of clauses 83 to 96 in the preparation of pentafluoroethyl copper complexes.
112. The use according to clause 111, wherein the complexes comprise one or more ligands, each of which comprises one or more atoms of group V.
113. The use according to clause 112, wherein the complexes have one ligand.
114. The use according to clause 112, wherein the complexes have two ligands.
115. The use according to clause 112, wherein the complexes have three ligands.
116. The use according to any one of clause 112 to 115, wherein the atoms of group V are selected from P and N.
117. The use according to clause 116, wherein the one or more ligands is a phosphine or a phosphite.
118. The use according to clause 117, wherein the one or more ligands is a phosphine.
119. The use according to clause 118, wherein the one or more ligands is selected from triphenylphosphine, tri(*n*-butyl)phosphine, tri(cyclohexylphosphine), tri(*p*-tolyl)phosphine and mixtures thereof.
120. The use according to clause 119, wherein the one or more ligands is triphenylphosphine.
121. The use according to any one of clause 112 to 116, wherein the one or more ligand is a heteroaromatic ring containing nitrogen.
122. The use according to clause 121, wherein the ligand is selected from the group consisting of: pyrrole, pyridine, oxazole, isoxazole, isoxazine, imidazole, pyrazole, pyridazine, pyrimidine, triazole, indole, benzimidazole, indazole, quinoline, isoquinoline, quinolizine, benzoxazine, benzodiazine, 2,2'-bipyridine, quinoxaline, quinazoline, phthalazine, naphthyridine, pteridine, acridine, phenazine and 1,10-phenanthroline.
123. The use according to clause 122, wherein the one or more ligands is selected from the group consisting of pyridine, 2,2'-bipyridine, 1,10-phenanthroline, quinoline, and isoquinoline.
124. The use according to clause 123, wherein the one or more ligands is selected from the group consisting of pyridine, 2,2'-bipyridine and 1,10-phenanthroline.
125. The use according to clause 111, wherein the pentafluoroethyl copper complex comprises the moiety [Cu(C₂F₅)X] wherein X is selected from (PR³₃)₂, (PR³₃)_{3,} L and (PR³₃)(L), wherein R³ is selected from the group consisting of: phenyl, *n*-butyl, cyclohexyl and *p*-tolyl; and L is a bidentate ligand containing at least two atoms of the group V.
126. The use according to clause 125, wherein L is selected from 2,2'-bipyridine and 1,10-phenanthroline.
127. The use according to clause 111, wherein the pentafluoroethyl copper complex comprises the moiety [Cu(C₂F₅)(PPh₃)₂].
128. The use according to clause 127 wherein the pentafluoroethyl copper complex is selected from the complexes of formula [Cu(C₂F₅)(PPh₃)₃], [Cu(C₂F₅)(PPh₃)₂] and mixtures thereof.
129. The use according to clause 111, wherein the ligand is a N-heterocyclic carbene.
130. A pentafluoroethyl copper complex obtainable according to any of clauses 111 to 129.

### References

Andrzejewska 2002 - Andrzejewska, M. et al. Eur. J. Med. Chem. 2002, 37, 973. Johansson 2003 - Johansson, A. et al. Bioorg. Med. Chem. 2003, 11, 2551
Carr 1988 - Carr, G.E., et al., J. Chem. Soc., Perkin Trans. 1 1988, 921 Freskos 1988 - Freskos, J.N., Synth. Commun. 1988, 18, 965
Urata 1991 - Urata, H., Fuchikami, T., Tetrahedron Lett. 1991, 32, 91
Kremlev 2010 - Kremlev, M.M., et al., J. Fluorine Chem. 2010, 19, 2063
Popov 2011 - Popov, I., et al., J. Am. Chem. Soc. 2011, 133, 9286-9289 (dx.doi.org/10/1021/ja2041942)
Zanardi 2011 - Zanardi, A., et al., J. Am. Chem. Soc. 2011, 133, 20901-20913 (dx.doi.org/10/1021/ja2081026)

## Claims

1. A method of obtaining a pentafluoroethylating composition comprising reacting a cuprating agent comprising the [Cu(OR¹)(OR²)] moiety with pentafluoroethane, wherein:
Cu is in the oxidation state of +1; and
R¹ is C₁₋₂₀ alkyl or C₅₋₂₀ aryl, wherein C₁₋₂₀ alkyl is optionally substituted with one or more groups selected from: C₅₋₂₀ aryl, C₁₋₂₀ alkoxy and C₅₋₂₀ aryloxy, and wherein C₅₋₂₀ aryl is optionally substituted with one or more groups selected from: C₁₋₂₀ alkyl, C₁₋₂₀ alkoxy, C₅₋₂₀ aryl, C₅₋₂₀ aryloxy, halo, nitro and cyano; and
R² is C₁₋₂₀ alkyl or C₅₋₂₀ aryl, wherein C₁₋₂₀ alkyl is optionally substituted with one or more groups selected from: C₅₋₂₀ aryl, C₁₋₂₀ alkoxy and C₅₋₂₀ aryloxy, and wherein C₅₋₂₀ aryl is optionally substituted with one or more groups selected from: C₁₋₂₀ alkyl, C₁₋₂₀ alkoxy, C₅₋₂₀ aryl, C₅₋₂₀ aryloxy, halo, nitro and cyano.

2. The method according to claim 1, wherein R¹ and R² are the same and unsubstituted C₁₋₇ alkyl.

3. The method according to either claim 1 or claim 2 that is carried out in an anhydrous aprotic solvent.

4. The method according to any one of the preceding claims wherein the cuprating agent comprising the [Cu(OR¹)(OR²)] moiety is a compound of formula (**II**) or a polymer or oligomer thereof:
MₗSₘCuₓ(OR¹)_{y}(OR₂)_{z}X_{q} (**II**)
wherein:
M is selected from the group consisting of lithium, sodium, potassium, rubidium, cesium, calcium, barium and magnesium; and
S is selected from N,N-dimethylformamide (DMF), 1,3-dimethyl-2-imidazolidinone (DMI), N,N-dimethylacetamide (DMAC), 1,3-dimethyl-3,4,5,6-tetrahydro-2(1H)-pyrimidinone (DMPU), N-methylpyrrolidone (NMP), dimethylsulfoxide (DMSO), tetramethylurea (TMU),
and hexamethylphosphoramide (HMPA);
R¹ and R² are as defined in claim 1;
X is chloride, bromide or iodide;
I is an integer from 1 to 20;
m is an integer from 0 to 20;
x, y and z are each an integer from 1 to 20;
q is an integer from 0 to 10; and where:
the sum of I and x is equal to the sum of y, z and q when M is selected from lithium, sodium, potassium, rubidium and cesium; or
the sum of twice the value of I and x is equal to the sum of y, z and q when M is selected from calcium, magnesium and barium.

5. The method according to claim 4, wherein the cuprating agent is selected from [K₈Cu₆(*tert*-BUO)₁₂(DMF)₈I]I; K(DMF)[Cu(*tert*-BUO)₂]; Na(DMF)₂[Cu(*tert*-BuO)₂] and polymers or oligomers thereof.

6. The method of any one of the preceding claims further comprising the step of treating the pentafluoroethylating composition obtained by reacting the cuprating agent with pentafluoroethane with an acid.

7. The method according to claim 6, wherein the acid is selected from hydrogen fluoride, hydrogen chloride, acetic acid, trifluoroacetic acid, formic acid, hydrogen fluoride pyridine, mixtures of compounds of formula NR³R⁴R⁵ with hydrogen fluoride, melamine/HF, poly[4-vinylpyridinium poly(hydrogen fluoride)], mixtures thereof and B(OR⁶)₃; wherein R³, R⁴, R⁵ and R⁶ are independently unsubstituted C₁₋₂₀ alkyl.

8. A pentafluroethylating composition obtainable by the method according to any one of claims 1 to 7.

9. A pentafluroethylating composition comprising the [Cu(C₂F₅)(OR¹)] moiety, wherein R¹ is C₁₋₂₀ alkyl or C₅₋₂₀ aryl, wherein C₁₋₂₀ alkyl is optionally substituted with one or more groups selected from: C₅₋₂₀ aryl, C₁₋₂₀ alkoxy and C₅₋₂₀ aryloxy, and wherein C₅₋₂₀ aryl is optionally substituted with one or more groups selected from: C₁₋₂₀ alkyl, C₁₋₂₀ alkoxy, C₅₋₂₀ aryl, C₅₋₂₀ aryloxy, halo, nitro and cyano.

10. The pentafluoroethylating composition of claim 9 that is a compound of formula (**III**) or a polymer of oligomer thereof:
M_{l'}S_{m'}Cu_{x'}(OR')_{y'}(C₂F₅)_{z'}X_{q'} (**III**)
wherein:
Cu is in the oxidation state +1;
M, S, R¹ and X are as defined in any one of claims 1 to 5;
I' is an integer from 1 to 20;
m' is an integer from 0 to 20;
x', y' and z' are each an integer from 1 to 20;
q' is an integer from 0 to 10; and where:
the sum of I' and x' is equal to the sum of y', z' and q' when M is selected from lithium, sodium, potassium, rubidium and cesium; or, alternatively,
the sum of twice the value of I' and x' is equal to the sum of y', z' and q' when M is selected from calcium, magnesium and barium.

11. The pentafluoroethylating composition according to claim 10 wherein
M is selected from sodium, potassium, and cesium;
q' is 0;
m' is an integer from 0 to 5;
l' is 1; and
x', y' and z' are 1.

12. The pentafluoroethylating composition according to claim 10 wherein compound of formula (III) is K(DMF)₂Cu(O-*tert*-Bu)(C₂F₅), a polymer or an oligomer thereof.

13. Use of a pentafluoroethylating composition as defined in any one of claims 8 to 12 in the transfer of a pentafluoroethyl group to a substrate selected from an electrophile, a terminal alkyne, or to the carbon atom of an organoboron compound.

14. The use of the pentafluoroethylating composition as defined in any one of claims 8 to 12 in the preparation of pentafluoroethyl copper complexes.

15. The use according to claim 14, wherein the pentafluoroethyl copper complex formed comprises Cu(C₂F₅)(PPh₃)₂.
